# EUROPEAN PATENT APPLICATION

(11) **EP 4 574 082 A1**
(43) Date of publication of application: **25.06.2025**
(21) Application number: 23854154.4
(22) Date of filing: 07.07.2023
(51) Int. Cl.: A61B 34/37, A61B 34/30, B25J 9/00, B25J 13/00

(54) **CATHETER ROBOT, CONTROL METHOD THEREFOR, AND MEDIUM**

(30) Priority: 18.08.2022 CN 202211002574; 30.09.2022 CN 202211214655; 30.09.2022 CN 202211207591
(71) Applicant: Shenzhen Edge Medical Co., Ltd., Shenzhen, Guangdong 518000 (CN)
(72) Inventor: WANG, Jiamian, Shenzhen, Guangdong 518000 (CN); WANG, Pai, Shenzhen, Guangdong 518000 (CN); GAO, Yuanqian, Shenzhen, Guangdong 518000 (CN)
(74) Representative: Metida
(86) International application number: PCT/CN2023/106450
(87) International publication number: WO 2024/037249

(57) **Abstract**

The present application discloses a catheter robot, a control method therefor, and a medium. The catheter robot comprises: a manipulator assembly and a control apparatus. The control apparatus is coupled with the manipulator assembly and is configured to acquire the installation direction of a catheter instrument in the manipulator assembly; determine one of a first mapping relationship and a second mapping relationship as a target mapping relationship according to the installation direction; and control, on the basis of the target mapping relationship, a driving mechanism in the manipulator assembly to manipulate the motion of the catheter instrument in the target motion direction. The manipulator assembly is controlled to drive the catheter instrument according to the mapping relationship corresponding to the installation direction of the catheter instrument in the manipulator assembly, so that the influence that changing the installation direction of the catheter instrument has on the catheter control effect is overcome, and the catheter robot can make adaptive adjustments according to the use environment.

## Description

### TECHNICAL FIELD

The present disclosure relates to medical apparatus and instruments technologies field, in particular to a catheter robot, a control method therefore, and medium.

### BACKGROUND

With the development of science and technology and the innovation of artificial intelligence technology, surgical robots have been widely used in surgical clinic, the surgical robots can replace doctors for on-site surgery, and doctors only need to operate and control the surgical robots on a remote console, so as to serve patients in more areas, such as remote areas lacking experienced surgeons.

The application of catheter robots is also gradually extensive. The catheter robot is equipped with a catheter at the end of the robot arm, which can enter the patient's body from the mouth, nasal cavity, etc., to perform internal examinations such as endoscopy or surgery. The catheter robot is generally set to the left and right arms, and the catheter installed on the left and right robot arms is divided into internal catheter and external catheter, and the internal catheter needs to be inserted into the external catheter, and finally inserted into the mouth and other parts of the human body through the introducer, so the catheter instruments installed with internal and external catheters can not be mixed, nor can they exchange directions at will.

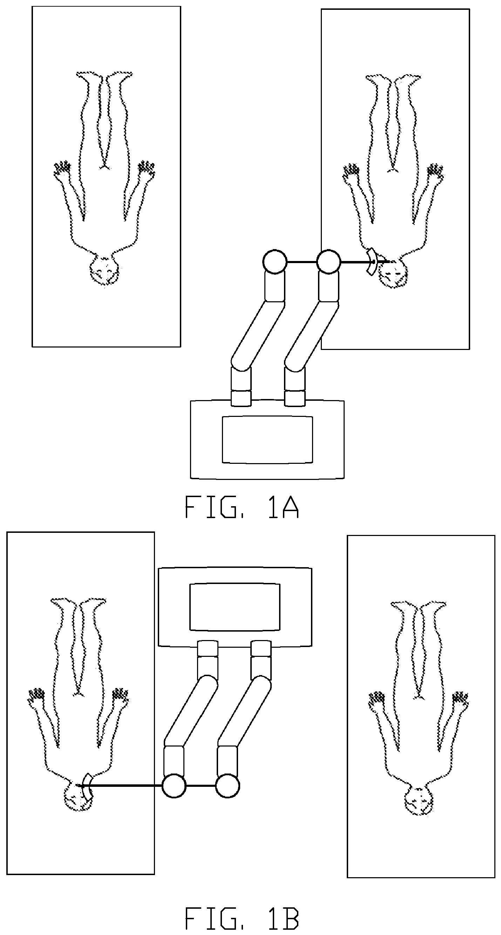

In the prior art, the working direction of the catheter robot is manually set. As shown in FIG. 1A, when the right arm of the catheter robot is configured as an external catheter instrument, the working direction is set to the right from the manual, that is, surgery can only be performed for patients in the right bed of the catheter robot, but not for patients in the left bed. In other words, if the bed of the patient in the next surgery is replaced to the left side of the catheter robot, the catheter robot needs to be moved (FIG. 1B shows the scene after 180° rotation), as the overall equipment of the catheter robot is heavy, it takes time and effort to move, and it also needs to reserve more space for the catheter robot to move, which requires higher space for the environment.

### SUMMARY OF THE INVENTION

In order to solve the problem that the catheter robot cannot automatically set the working direction in the prior art, resulting in the need to move the catheter robot when changing the direction of the surgical bed, which makes the operation time-consuming and laborious and has high spatial requirements on the environment, the present disclosure provides the following technical solutions:

One aspect of the present disclosure provides a catheter robot including:
a manipulator assembly, the manipulator assembly configured to install and drive a catheter instrument; and
a control apparatus, the control apparatus coupled with the manipulator assembly and configured to:
acquire an installation direction of the catheter instrument in the manipulator assembly;
determine a target mapping relationship according to the installation direction;
wherein the target mapping relationship comprises a first mapping relationship and a second mapping relationship, the first mapping relationship comprises a mapping relationship between a driving mechanism in the manipulator assembly and a target motion direction when the installation direction being a first direction is associated, the second mapping relationship comprises a mapping relationship between the driving mechanism in the manipulator assembly and the target motion direction when the installation direction being a second direction is associated;
control, based on the target mapping relationship, the driving mechanism in the manipulator assembly to manipulate a motion of the catheter instrument in the target motion direction.

Another aspect of the present disclosure provides a control method of catheter robot, the catheter robot including:
a manipulator assembly, the manipulator assembly configured to install and drive a catheter instrument; and
a control apparatus, the control apparatus coupled with the manipulator assembly and configured to:
acquire an installation direction of the catheter instrument in the manipulator assembly;
determine a target mapping relationship from one of a first mapping relationship and a second mapping relationship according to the installation direction, the target mapping relationship comprises a first mapping relationship and a second mapping relationship, the first mapping relationship comprises a mapping relationship between a driving mechanism in the manipulator assembly and a target motion direction when the installation direction being a first direction is associated, the second mapping relationship comprises a mapping relationship between the driving mechanism in the manipulator assembly and the target motion direction when the installation direction being a second direction is associated;
control, based on the target mapping relationship, the driving mechanism in the manipulator assembly to manipulate a motion of the catheter instrument in the target motion direction.

Another aspect of the present disclosure provides a readable storage medium, a computer program is stored on the readable storage medium, and when the computer program is executed by a processor, the steps of the control method of catheter robot are implemented.

The beneficial effect of the present disclosure is that the manipulator assembly is controlled to drive the catheter instrument according to the installation direction of the catheter instrument in the manipulator assembly with the mapping relationship corresponding to the installation direction, thereby solving the impact on the catheter control effect caused by the change of the installation direction of the catheter instrument, so that the catheter robot can make adaptive adjustments according to the use environment.

### BRIEF DESCRIPTION OF THE DRAWINGS

FIG. 1A is a schematic diagram of a use scenario of a catheter robot in the prior art;
FIG. 1B is a schematic diagram of a use scenario of the catheter robot after movement in the prior art;
FIG. 2 is a structural schematic diagrams of an embodiment of the present application of the catheter robot.
FIG. 3 is a structural schematic diagram of an embodiment of the present application of an installation plate.
FIG. 4 is a structural schematic diagram of an embodiment of the present application of an installation box.
FIG. 5 is a structural schematic diagram of an embodiment of the present application when the installation direction of the installation box is correct;
FIG. 6 is a structural schematic diagram of an embodiment of the present application when the installation direction of the installation box is incorrect;
FIG. 7 is a structural schematic diagram of a mapping relationship between a driving plate and a transmission plate of an embodiment of an external sheath instrument installed on a left mechanical arm of the present application;
FIG. 8 is a structural schematic diagram of a mapping relationship between the driving plate and the transmission plate of an embodiment of an internal tube instrument installed on the left mechanical arm of the present application;
FIG. 9 is a structural schematic diagram of a mapping relationship between the driving plate and the transmission plate before rotation of an embodiment of the internal tube instrument installed on the left mechanical arm of the present application;
FIG. 10 is a structural schematic diagram of a mapping relationship between the driving plate and the transmission plate after rotation of an embodiment of the internal tube instrument installed on the left mechanical arm of the present application;
FIG. 11 is a flow chart of an embodiment of the present application of a control method;
FIG. 12 is a flow chart of an embodiment of the present application of the control method;
FIG. 13 is a flow chart of an embodiment of the present application of the control method;
FIG. 14 is a flow chart of an embodiment of the present application of the control method;
FIG. 15 is a structural schematic diagram of a mapping relationship between the driving plate and the transmission plate of an embodiment of the external sheath instrument installed on a right mechanical arm of the present application;
FIG. 16 is a structural schematic diagram of a mapping relationship between the driving plate and the transmission plate of an embodiment of the internal tube instrument installed on the right mechanical arm of the present application;
FIG. 17 is a structural schematic diagram of a mapping relationship between the driving plate and the transmission plate before rotation of an embodiment of the internal tube instrument installed on the right mechanical arm of the present application;
FIG. 18 is a structural schematic diagram of a mapping relationship between the driving plate and the transmission plate after rotation of an embodiment of the internal tube instrument installed on the right mechanical arm of the present application;
FIG. 19 is a structural schematic diagram of an embodiment of the present application of controlling a bending direction of the catheter end;
FIG. 20 is a structural schematic diagram of an embodiment of the present application of controlling the bending direction of the catheter end;
FIG. 21 is a flowchart of an embodiment of the present application of the control method of catheter robot;
FIG. 22 is a structural schematic diagram of an embodiment of the present application of the catheter robot in a first direction;
FIG. 23 is a structural schematic diagram of an embodiment of the present application of the mechanical arm and a manipulator assembly of the catheter robot;
FIG. 24 is a structural schematic diagram of an embodiment of the present application of the catheter instrument when the catheter robot in the first direction;
FIG. 25 is a structural schematic diagram of an embodiment of the present application of the catheter robot in a second direction;
FIG. 26 is a structural schematic diagram of an embodiment of the present application of the catheter instrument when the catheter robot in the second direction;
FIG. 27 is a structural schematic diagram of another embodiment of the present application of the catheter robot in the first direction;
FIG. 28 is a structural schematic diagram of another embodiment of the present application of the catheter robot in the second direction;
FIG. 29 is a structural schematic diagram of another embodiment of the present application of the mechanical arm and the manipulator assembly of the catheter robot;
FIG. 30 is a structural schematic diagram of another embodiment of the present application of the catheter instrument when the catheter robot in the second direction;
FIG. 31 is a structural schematic diagram of an embodiment of the present application of the driving plate and the transmission plate of the catheter robot;
FIG. 32 is a schematic flow chart of an embodiment of the present application of a catheter zeroing method of the catheter robot;
FIG. 33 is a schematic flow chart of an embodiment of the present application of a zeroing process of the catheter robot;
FIG. 34 is a schematic flow chart of an embodiment of the present application of the catheter zeroing method of the catheter robot;
FIG. 35 is a schematic flow chart of an embodiment of the present application of the catheter zeroing method of the catheter robot.

### DETAILED DESCRIPTION

In order to facilitate the understanding of the present disclosure, the present disclosure is described in more detail below in conjunction with the accompanying drawings and specific embodiments. The preferred embodiments of the present disclosure are given in the accompanying drawings. However, the present disclosure can be implemented in many different forms and is not limited to the embodiments described in this specification. On the contrary, the purpose of providing these embodiments is to make the understanding of the disclosure of the present disclosure more thorough and comprehensive.

It should be noted that, unless otherwise defined, all technical and scientific terms used in this specification have the same meaning as those commonly understood by those skilled in the art in the technical field of the present disclosure. The terms used in the specification of the present disclosure are only for the purpose of describing specific embodiments and are not intended to limit the present disclosure. For example, the term "plurality" includes two or more.

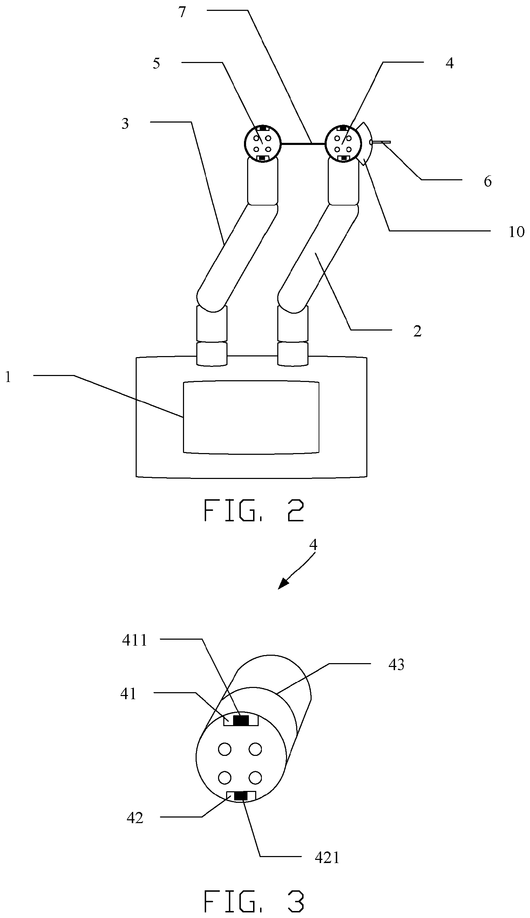

In one aspect, the present disclosure provides a control method of a catheter robot. Referring to FIGS. 2 to 5, the catheter robot includes a manipulator assembly installed on a trolley 1, the manipulator assembly comprises a mechanical arm and an installation plate disposed at the end of the mechanical arm. As shown in FIG. 2, ends of the two mechanical arms (the first mechanical arm 2 and the second mechanical arm 3) are respectively provided with the installation plates, and the installation plates are used to install the catheter instrument, the installation plates include a first installation plate 4 located at the end of the first mechanical arm 2 and a second installation plate 5 located at the end of the second mechanical arm 3, the catheter instrument includes a first catheter instrument 8 and a second catheter instrument 9, the first installation plate 4 is used to install the first catheter instrument 8 or the second catheter instrument 9, the second installation plate 5 is used to install the second catheter instrument 9 or the first catheter instrument 8, the types of catheter instruments include an internal tube instrument and an external sheath instrument, the first catheter instrument 8 and the second catheter instrument 9 can be of the same type or different types, when both are used in the same catheter robot, they are usually catheter instruments of different types. The internal tube instrument is connected with an internal catheter 7, and the external sheath instrument is connected with an external catheter 6. During surgery, the internal tube instrument is inserted into the external tube instrument in the same direction, that is, the internal catheter 7 is inserted into the external catheter 6 in the same direction for use.

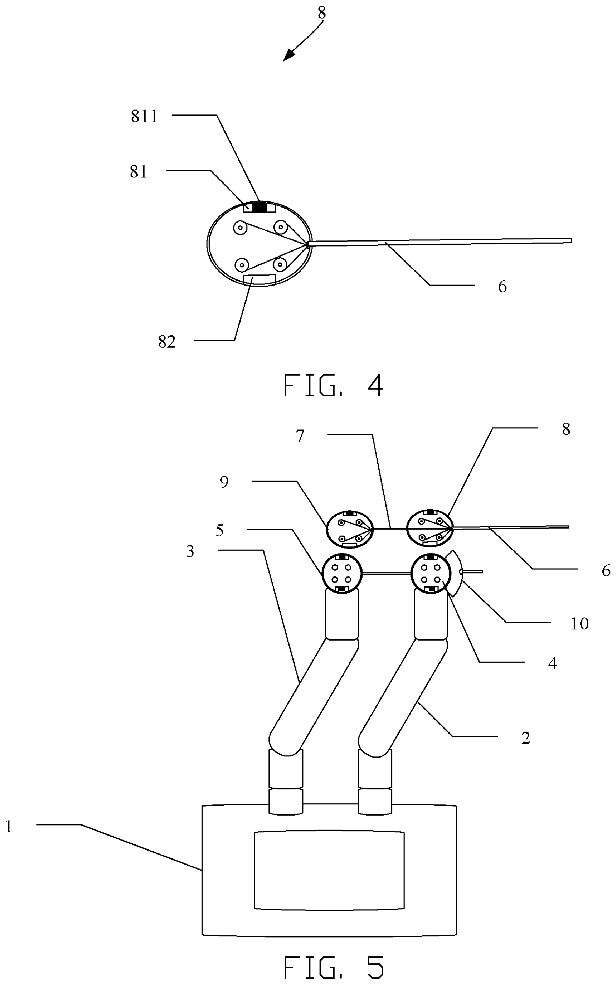

Referring to FIG. 3, the installation plate is provided with identifying devices 411 and 421. Referring to FIG4 , the catheter instrument is provided with an identification device 811, the identifying device 411 is used to detect the identification device 811 to obtain identification information, and determine whether the catheter instrument 8 is the internal tube instrument or the external sheath instrument according to the identification information. Exemplarily, the information carried by the identification device on different catheter devices is different, and the information can uniquely identify the type of the catheter device.

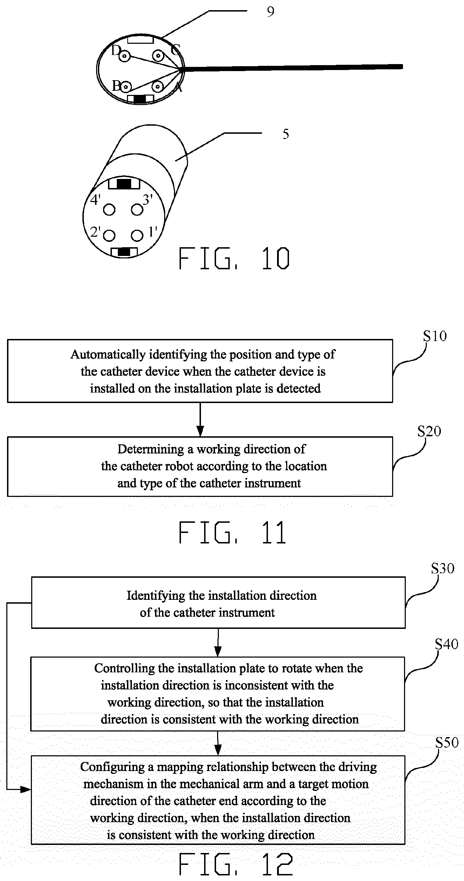

Referring to FIG. 11, the control method of the catheter robot of the present application includes the following steps:
S10: automatically identifying the position and type of the catheter device when the catheter device is installed on the installation plate is detected;
S20: determining a working direction of the catheter robot according to the location and type of the catheter instrument.

Exemplarily, when the catheter instrument 8 is installed on the installation plate 4, the identifying device 411 on the installation plate 4 contacts the identification device 811 on the catheter instrument 8, or the distance between the two reaches its threshold, so that the identifying device 411 can detect the identification device 811 and obtain the identification information.

The identifying device 411 and the identification device 811 can be implemented using sensors, including contact sensors and non-contact sensors. For example, the contact sensor can be a plug-in matching form of an interface and a plug, while the non-contact sensor can be an electromagnetic, near-field sensor, etc. In other embodiments, the identifying device can also be replaced by an imaging device, which is configured to at least capture images of the installation plates at the ends of the two mechanical arms of the catheter robot, when the catheter instrument is installed on the installation plate, the position and type of the catheter instrument can be automatically identified based on the images captured by the imaging device.

The position of the catheter instrument refers to whether the catheter instrument is installed on the first mechanical arm 2 or the second mechanical arm 3. The acquired identification information may include the position of the catheter instrument. Exemplarily, when the identifying device 411 or 421 detects the identification device 811, it can be identified that the catheter instrument is installed on the first mechanical arm 2.

After the identification information is acquired, the type of the catheter instrument 8 is automatically identified. Specifically, the identification information can be transmitted to the control apparatus of the catheter robot to identify the type of the catheter instrument. The control apparatus is located inside the catheter robot trolley 1, or inside a main console apparatus connected to the catheter robot trolley 1. The computer program for identifying the type of catheter instrument can be executed in the control apparatus, or can be a control program or service for remote communication in the cloud.

Further, the working direction of the catheter robot is determined according to the position and type of the installed catheter instrument 4, so that the catheter robot can switch between different working directions.

For example, when the external sheath instrument is installed on the right mechanical arm of the catheter robot (the first mechanical arm 2 as shown in FIG. 5), the working direction of the catheter robot is determined to be rightward. When the next operation requires the catheter robot to work to the left, the external sheath instrument only needs to be installed on the left mechanical arm of the catheter robot (the second mechanical arm 3 as shown in FIG. 5), which is automatically recognized by the catheter robot, thereby redetermining the working direction of the catheter robot to be leftward, thereby achieving the effect of performing the next operation without having to move the spatial position of the catheter robot or manually set the working direction of the catheter robot.

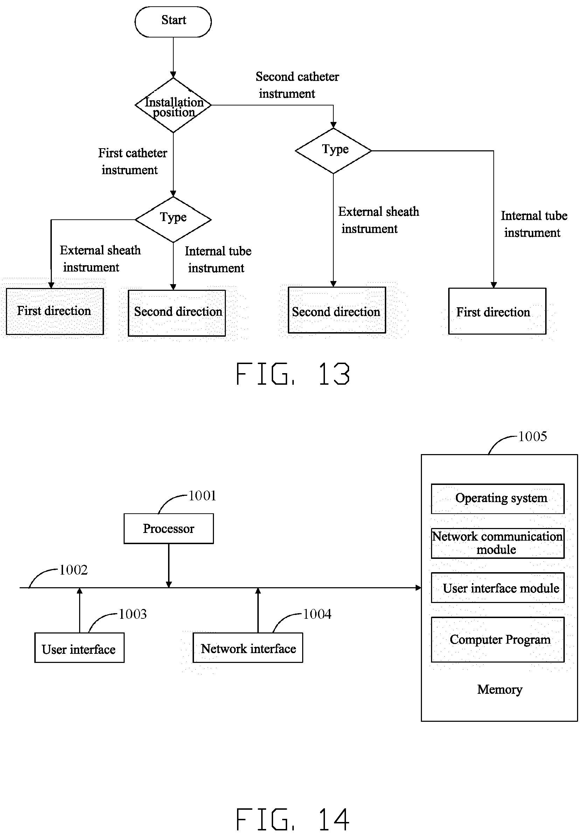

Further, referring to FIG. 13, the working direction includes a first direction and a second direction, and the position of the catheter instrument includes a first position and a second position, the first position corresponds to the first catheter instrument, and the second position corresponds to the second catheter instrument; determining the working direction of the catheter robot according to the position and type of the catheter instrument also includes:

If the position of the catheter instrument is the first position and the type of the first catheter instrument 8 is an external sheath instrument, the working direction of the catheter robot is determined to be the first direction; if the position of the catheter instrument is the first position and the type of the first catheter instrument 8 is an internal tube instrument, the working direction of the catheter robot is determined to be the second direction; if the position of the catheter instrument is the second position, and the type of the second catheter instrument 9 is an external sheath instrument, the working direction of the catheter robot is determined to be the second direction; if the position of the catheter instrument is the second position, and the type of the second catheter instrument 9 is an internal tube instrument, the working direction of the catheter robot is determined to be the first direction.

Preferably, the first direction and the second direction are horizontally opposite directions, for example, the first direction corresponds to the right, and the second direction corresponds to the left. As shown in FIG. 5, the first mechanical arm 2 is a right mechanical arm, and the second mechanical arm 3 is a left mechanical arm. When the first catheter instrument is installed on the right mechanical arm of the catheter robot, and the second catheter instrument is installed on the left mechanical arm of the catheter robot: if the type of the first catheter instrument installed on the right mechanical arm is the external sheath instrument, the working direction of the catheter robot is determined to be the first direction, i.e., rightward; if the type of the first catheter instrument installed on the right mechanical arm is the internal tube instrument, the working direction of the catheter robot is determined to be the second direction, i.e., leftward; if the second catheter instrument 9 installed on the left mechanical arm is the external sheath instrument, the working direction of the catheter robot is determined to be the second direction, i.e., leftward; if the second catheter instrument 9 installed on the left mechanical arm is the internal tube instrument, the working direction of the catheter robot is determined to be the first direction, i.e., rightward. Specifically referring to FIG. 5, FIG. 5 shows a specific embodiment of the catheter robot, wherein the working direction is rightward, the right mechanical arm (i.e., the first mechanical arm 2) is installed with the external sheath instrument, and the left mechanical arm (i.e., the second mechanical arm 3) is installed with the internal tube instrument.

Further, after the working direction of the catheter robot is determined, a prompt message is issued to remind the user of the determined working direction, so that the user can further confirm whether the working direction needs to be changed.

Further, after the first catheter instrument 8 and the second catheter instrument 9 are fully installed, if it is determined that the first catheter instrument 8 and the second catheter instrument 9 are of the same type, an alarm message is issued to prompt the user to replace the catheter instrument.

Exemplarily, an installation sequence may be to install the first catheter instrument 8 first and then the second catheter instrument 9, or to install the second catheter instrument 9 first and then the first catheter instrument 8, or to install the first catheter instrument 8 and the second catheter instrument 9 at the same time.

For example, when the first catheter instrument 8 is installed first, the prompt message is issued after the first catheter instrument 8 is installed, including prompting the user to install the second catheter instrument 9.

As described in the above embodiment, referring to FIG. 5, when the first catheter instrument 8 is installed on the right mechanical arm (as shown in FIG. 5, the first catheter instrument 8 is installed on the first installation plate 4), and the type of the first catheter instrument 8 is the external sheath instrument, the working direction is determined to be rightward. At this time, the surgeon or nurse is prompted that the working direction of the catheter robot is to perform surgery on the right side, but the surgeon or nurse finds that the patient is actually on the left side of the catheter robot, indicating an installation error, the surgeon or nurse can then remove the first installed catheter instrument and reinstall it.

Reinstallation includes two situations: 1. Replace the type of the new catheter instrument and install it to the first installation plate 4 (replace it with the internal tube instrument); 2. Install the removed original external sheath instrument to the second installation plate 5 on the left.

When the second reinstallation situation is performed, the first catheter instrument 8 installed earlier is replaced with the second catheter instrument 9.

After reinstallation, the prompt message is issued to remind the surgeon or nurse that the working direction of the catheter robot is to perform surgery on the left side, and the surgeon or nurse can continue to install the first catheter instrument 8 (right mechanical arm). After the first catheter instrument 8 is installed, if it is determined that the first catheter instrument 8 and the second catheter instrument 9 are of the same type (both are internal tube instruments), the alarm message is issued to remind the user to replace the catheter instrument.

When the second catheter instrument 9 is installed first, the process of subsequent prompt message is similar to the above embodiment, which will not be repeated here.

When the first catheter instrument 8 and the second catheter instrument 9 are installed at the same time, the working direction of the catheter robot is determined according to the type of the first catheter instrument 8 or the second catheter instrument 9. If the first catheter instrument 8 and the second catheter instrument 9 are of the same type, the working direction cannot be determined, and the alarm message is directly issued to prompt the user to replace the catheter instrument.

Further, a rotating device for driving the installation plate to rotate around the central axis of the installation plate is provided at a distal end of the mechanical arm, the rotation of the installation plate can be controlled by driving the rotating device to move. Referring to FIG. 12, further, after determining the working direction of the catheter robot, the control method further includes:
S30: identifying the installation direction of the catheter instrument;
S40: controlling the installation plate to rotate when the installation direction is inconsistent with the working direction, so that the installation direction is consistent with the working direction.

Through the above method, when the user installs incorrectly, the catheter instrument can be automatically identified and corrected to the correct position without the need for manual operation by the user, thereby further improving efficiency and enhancing user experience.

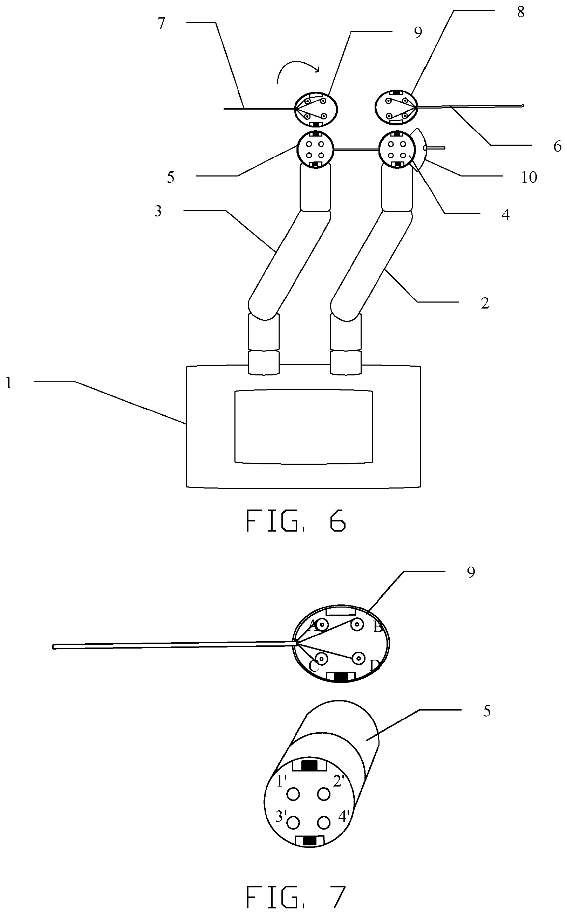

Exemplarily, referring to FIG. 5 and FIG. 6, FIG. 5 is a case where the catheter instrument is correctly installed, and FIG. 6 is a case where the catheter instrument is incorrectly installed. In FIG. 5, the second catheter instrument installed by the left mechanical arm is the internal tube instrument, and the first catheter instrument installed by the right mechanical arm is the external sheath instrument, the working direction of the catheter robot is rightward, and the installation directions of the first catheter device 8 and the second catheter device 9 are both rightward. The internal catheter is inserted into the external catheter through the external sheath instrument on the right, and then inserted into the human oral cavity and other parts via the right introducer. The difference between FIG. 6 and FIG. 5 is that the second catheter instrument installed by the left mechanical arm in FIG. 6 is the internal tube instrument, and the correct direction of the internal tube instrument should be the working direction (rightward), but the installation direction of the internal tube instrument is leftward, resulting in the internal tube instrument being unable to be inserted into the external tube through the first catheter instrument, thus causing installation errors. At this time, the second catheter instrument is rotated 180° to the working direction by the rotating device, so that the internal tube is rotated from leftward to rightward, thereby completing the correct installation.

Preferably, referring to FIG. 4, the rotating device is provided on the installation plate, and the device may be electrically driven or mechanically driven. The present application does not limit the implementation. The rotation angle may be n*360°+180°, where n is an integer greater than or equal to 0, and the installation plate only needs to be switched in two opposite directions.

Preferably, referring to FIG. 3 and FIG. 4, each installation plate has a first installation bayonet 41 and a second installation bayonet 42 symmetrically arranged, and each catheter instrument has a first installation interface 81 and a second installation interface 82 symmetrically arranged, which are mechanically engaged with the installation bayonet to complete the installation and fixation of the catheter instrument; the first identifying device 411 is installed in the first installation bayonet 41, the second identifying device 421 is installed in the second installation bayonet 42, and each catheter instrument is equipped with an identification device 811, which is set in an installation interface of the catheter instrument;
identifying the installation direction of the catheter instrument includes:
determining the installation direction as the first direction if the first identifying device 411 identifies the identification device 811;
determining the installation direction as the second direction if the second identifying device 421 identifies the identification device 811.

Exemplarily, the installation plate in FIG. 3 has the first installation bayonet 41 and the second installation buckle 42 that are symmetrical up and down, the first installation bayonet 41 is provided with the first identifying device 411, and the second installation bayonet 42 is provided with a second identifying device 421.

FIG. 4 shows the external sheath instrument with the external catheter 6 connected to the right side, the external sheath instrument corresponds to the first installation interface 81 and the second installation interface 82 that are symmetrical in upper and lower directions, the identification device 811 is arranged in the first installation interface 81. In other embodiments, the identification device 811 can also be installed in the second installation interface 82, which is not limited here.

When the external sheath instrument in FIG. 4 is installed on the installation plate 4 in FIG. 3, if the first identifying device 411 on the upside of the installation plate 4 identifies the identification device 811, then can determine that the installation direction of this external sheath instrument is rightward, and its external catheter 6 is also rightward. On the contrary, if the second identifying device 421 on the downside of the installation plate 4 identifies the identification device 811, then can determine that the installation direction of the external sheath instrument is leftward, and its external catheter 6 is also leftward.

In other embodiments, the first identifying device 411 and the second identifying device 421 can also be symmetrically arranged on the left and right sides of the installation plate 4, instead of being arranged in the first installation bayonet 41 and the second installation bayonet 42 symmetrically in the above embodiment.

Further, the control method further includes:
When it is detected that the catheter instrument is removed, controlling the installation plate to automatically rotate to the initial position. This reset operation can make it less prone to errors during the next installation and also reduce the complexity of the control program when detecting the direction.

Further, referring to FIG. 12, after identifying the installation direction of the catheter instrument, the control method further includes:
S50: configuring a mapping relationship between the driving mechanism in the mechanical arm and a target motion direction of the catheter end according to the working direction, when the installation direction is consistent with the working direction.

Further, after controlling the installation plate to rotate, configuring the mapping relationship between the driving mechanism in the mechanical arm and a target motion direction of the catheter end according to the working direction. This is because after the installation plate rotates, the mapping relationship between the driving mechanism and the target motion direction of the catheter end changes and needs to be reconfigured.

Specifically, in the catheter robot, the motion of the catheter end can be decoupled into two parts of motion, one is a feed motion, and the other is a bending motion. The feed motion refers to the advancement and retreat of the catheter end in the human body cavity, which is specifically achieved by the forward and backward motion of the mechanical arm to pull the catheter forward and backward, and is mainly used for advancement and retreat; the bending motion refers to the up, down, left, and right bending of the catheter end in a plane perpendicular to the feed motion direction, and is mainly used for adjusting an angle of the catheter end, and is specifically driven by the driving plate on the installation plate.

Therefore, the feed motion originates from a driving of a driving joint in the mechanical arm, and the bending motion originates from a driving of the driving plate in the installation plate. Thus, the mapping relationship mainly refers to the relationship between the driving mechanism in the mechanical arm (including the driving joint in the mechanical arm and the driving plate in the installation plate) and the target motion direction of the catheter end, the target motion direction includes a target bending motion direction and a target feed motion direction.

Further, the installation plate includes a plurality of driving plates, the catheter instrument includes a plurality of transmission plates, the driving plates are removably connected to the transmission plates, and the catheter end is driven to move through the transmission plates, the driving mechanism includes a plurality of driving plates, the target motion direction includes a target bending motion direction of the catheter end, and the mapping relationship between the driving mechanism in the mechanical arm and the target motion direction of the catheter end configured according to the working direction includes:

Configuring a mapping relationship between the driving plate and the target bending motion direction according to the working direction and a physical connection correspondence between the driving plate and the transmission plate.

Preferably, the installation plate is provided with the plurality of driving plates, for example, N pairs; and the catheter instrument is correspondingly provided with the plurality of transmission plates, for example, N pairs. In some embodiments, a pair of transmission plates may include a driving wire, two ends of which are respectively wound around two transmission plates, and the middle is wound around the catheter end. One of the two transmission plates rotates clockwise and the other rotates counterclockwise to achieve the coordinated control of the retraction and extension of the driving wire, so as to achieve the motion of the catheter end in the corresponding bending direction. a pair of transmission plates may include two driving wires, one end of one driving wire is wound around the transmission plate, and the other end is arranged at the catheter end, and one end of the other driving wire is wound around another transmission plate, and the other end is arranged at the catheter end. One of the two transmission plates rotates clockwise, and the other rotates counterclockwise to achieve the coordinated control of the retraction and extension of the driving wires, and can also realize the motion of the catheter end in the corresponding bending direction.

Exemplarily, referring to FIG. 7, the transmission plates A-D are provided with driving wires, which are connected in pairs to the internal catheter or the external catheter, and the driving plates 1'-4' drive the transmission plates A-D to rotate, thereby causing the paired driving wires to retract and release, and the internal catheter and the external catheter are pulled to adjust the angle at the far end. Therefore, the rotation direction of the driving plates 1'-4', as well as the parameters such as the rotation speed and the limit directly determine the adjustment of the internal and external catheters at the far end. Since the installation directions and types of the installation box 9 are different, if the same mapping relationship is used, the catheter robot can only work in one working direction. The method disclosed in the present invention can automatically set a new mapping relationship when the catheter robot switches the working direction, so that a variety of working directions of the catheter robot can be adapted without manual setting. Furthermore, the method disclosed in the present invention can also reset a new mapping relationship after detecting an installation error of the installation plate and performing rotation correction, without manual setting.

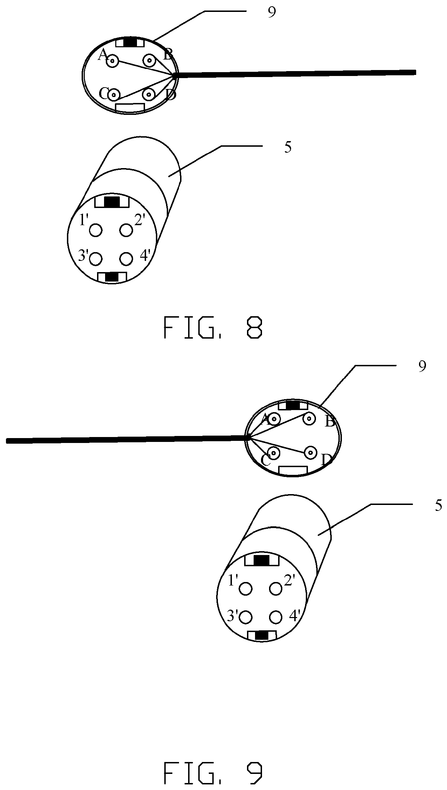

Exemplarily, referring to FIGS. 7 to 10, the installation directions of different types of catheter instruments installed on the second installation plate 5 (left mechanical arm) are shown, the external sheath instrument of the left mechanical arm can only work to the left, and the internal tube instrument of the left mechanical arm can only work to the right. Therefore, FIG. 7 shows that the external sheath instrument is installed to the left (correct installation), FIG. 8 shows that the internal tube instrument is installed to the right (correct installation), FIG. 9 shows that the internal tube instrument is installed to the right (before rotation, incorrect installation), and FIG. 10 shows that the internal tube instrument is installed to the right (after rotation, correct installation).

Each driving plate on the installation plate corresponds to each transmission plate in the catheter instrument, the driving plate and the transmission plate can be connected by, for example, a concave-convex matching structure, for example, the driving plate is provided with a convex column, and the transmission plate is provided with a groove matched with the convex column, and the two are plugged together.

In FIG. 7, the driving plate 1', the driving plate 2', the driving plate 3' and the driving plate 4' correspond to the transmission plate A, the transmission plate B, the transmission plate C and the transmission plate D respectively, wherein the transmission plate A and the transmission plate C control a pair of proximal driving wires of the external catheter, and the relative positions are A up and C down, and the transmission plate B and the transmission plate D control a pair of distal driving wires of the external catheter, and the relative positions are B up and D down, and their motion relationship moves according to the motion parameters of the catheter to the left;

In FIG. 8, the driving plate 1', the driving plate 2', the driving plate 3' and the driving plate 4' correspond to the transmission plate A, the transmission plate B, the transmission plate C and the transmission plate D respectively, wherein the transmission plate A and the transmission plate C control a pair of distal driving wires of the internal catheter, and the relative positions are A up and C down, and the transmission plate B and the transmission plate D control a pair of proximal driving wires of the internal catheter, and the relative positions are B up and D down, and their motion relationship moves according to the motion parameters of the catheter to the right;

In FIG. 9, the driving plate 1', the driving plate 2', the driving plate 3' and the driving plate 4' correspond to the transmission plate A, the transmission plate B, the transmission plate C and the transmission plate D respectively, wherein the transmission plate A and the transmission plate C control a pair of proximal driving wires of the internal catheter, and the relative positions are A up and C down, and the transmission plate B and the transmission plate D control a pair of distal driving wires of the internal catheter, and the relative positions are B up and D down, and their motion relationship moves according to the motion parameters of the catheter to the left;

In FIG. 10, the driving plate 1', the driving plate 2', the driving plate 3' and the driving plate 4' correspond to the transmission plate A, the transmission plate B, the transmission plate C and the transmission plate D respectively, wherein the transmission plate A and the transmission plate C control a pair of proximal driving wires of the internal catheter, and the relative positions are C up and A down, and the transmission plate B and the transmission plate D control a pair of distal driving wires of the internal catheter, and the relative positions are D up and B down, and their motion relationship moves according to the motion parameters of the catheter to the right.

Further, compared with FIG. 8, although the corresponding relationship between the driving plate and the transmission plate is consistent in FIG. 10, the position of the driving plate has rotated. Therefore, in addition to the motion direction of the driving plate needing to be adjusted, the motion speed and the like also need to be changed in accordance with the principle of central symmetry.

The above is only an embodiment of the mapping relationship, and there may be other ways to achieve this function, which are not limited here. For example, in other embodiments, it may be arranged in diagonal lines.

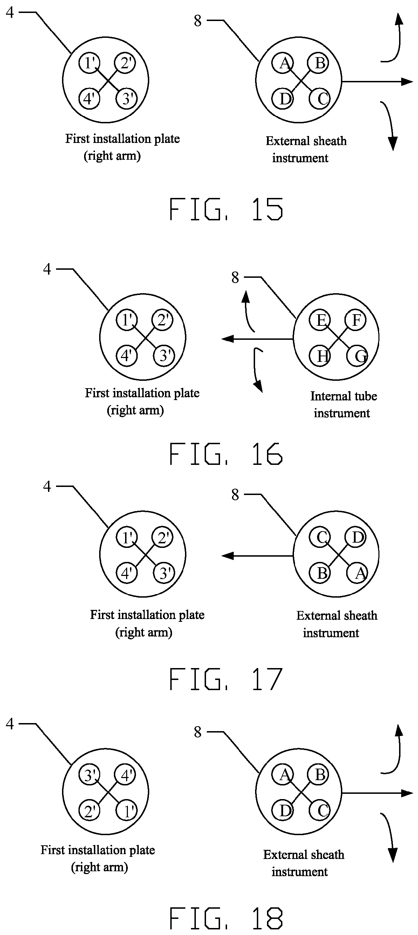

Exemplarily, referring to FIGS. 15 to 18, the installation directions of different types of catheter instruments installed on the first installation plate 4 (right mechanical arm) are shown, the external sheath instrument of the right mechanical arm must be facing right to work, and the internal tube instrument of the right mechanical arm must be facing left to work. Therefore, FIG. 15 shows that the external sheath instrument is installed to the right (correct installation), FIG. 16 shows that the internal tube instrument is installed to the left (correct installation), FIG. 17 shows that the external sheath instrument is installed to the left (before rotation, incorrect installation), and FIG. 18 shows that the external sheath instrument is installed to the right (after rotation, correct installation). The driving plates controlling a group of driving wires are arranged diagonally, as shown in FIG. 15, the driving plate 1' and the driving plate 3' on the first installation plate 4 are a pair.

In FIG. 15, assume that the driving plate 1' and the driving plate 3' are a pair controlling the bending in two directions on one degree of freedom, and the driving plate 2' and the driving plate 4' are a pair controlling the bending in two directions on another degree of freedom. Correspondingly, the transmission plate A and the transmission plate C are a pair controlling the bending in two directions on one degree of freedom, and the transmission plate B and the transmission plate D are a pair controlling the bending in two directions on another degree of freedom. The driving plate 1' is connected to the transmission plate A, the driving plate 2' is connected to the transmission plate B, the driving plate 3' is connected to the transmission plate C, and the driving plate 4' is connected to the transmission plate D correspondingly.

Different degrees of freedom can be directions that are perpendicular to each other, for example, one degree of freedom can be in the up and down directions (as on the paper), and the other degree of freedom can be in the inside and outside directions (as on the paper).

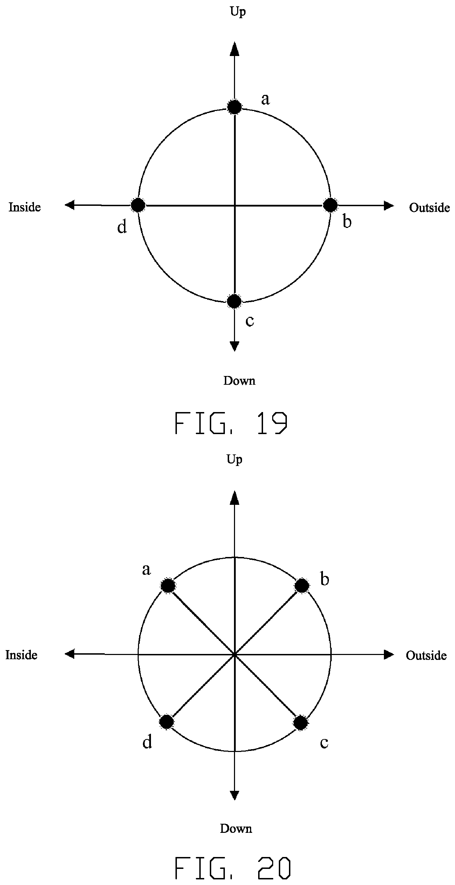

Specifically, the corresponding degree of freedom can be adjusted by arranging the retractable and unretractable connection relationship between the transmission plate and the catheter end. Referring to FIG. 19, the cross section of the catheter end is represented by the circumference, and the four points a-d intersecting with the circumference are connected to the transmission plate A-D by the driving wires. For example, one end of the driving wire is connected to point a in FIG. 19, and the other end is connected to the transmission plate A, the transmission plate A rotates by releasing the wire, thereby driving the driving wire to pull point a, so that the catheter end moves upward. Similarly, one end of another driving wire is connected to point c on the circumference, and the other end is connected to the transmission plate C, the transmission plate C rotates by releasing the wire, thereby driving the driving wire to pull point c, so that the catheter end moves downward. Accordingly, the transmission plates A and C can also be rotated to release the wire, and the catheter end can be bent upward by the transmission plate A and the catheter end can be bent downward by the transmission plate C and the catheter end can be bent downward. Similarly, the catheter end can be bent outward or inward by the cooperation of the transmission plate B and the transmission plate D. Therefore, FIG. 19 can realize the direction control of two degrees of freedom, one degree of freedom is the up and down directions, and the other degree of freedom is the inside and outside directions.

At this time, the end of the external catheter can be bent in different directions according to the driving of the driving plate, and the bending directions include upward, downward, inward, and outward. The mapping relationship can be as follows:
The drive plate 1' is retracted and the drive plate 3' is released: the external catheter is bent upward;
The drive plate 3' is retracted and the drive plate 1' is released: the external catheter is bent downward;
The drive plate 2' is retracted and the drive plate 4' is released: the external catheter is bent outward (perpendicular to the paper surface and outward);
The drive plate 4' is retracted and the drive plate 2' is released: the external catheter is bent inward (perpendicular to the paper surface and inward).

In FIG. 16, assume that the driving plate 1' and the driving plate 3' are a pair controlling the bending in two directions on one degree of freedom, and the driving plate 2' and the driving plate 4' are a pair controlling the bending in two directions on another degree of freedom. Correspondingly, the transmission plate E and the transmission plate G are a pair controlling the bending in two directions on one degree of freedom, and the transmission plate F and the transmission plate H are a pair controlling the bending in two directions on another degree of freedom. The driving plate 1' is connected to the transmission plate E, the driving plate 2' is connected to the transmission plate F, the driving plate 3' is connected to the transmission plate G, and the driving plate 4' is connected to the transmission plate H correspondingly.

Different degrees of freedom can be directions that are perpendicular to each other, for example, one degree of freedom can be in the up and down directions, and the other degree of freedom can be in the inside and outside directions.

At this time, the end of the internal catheter can be bent in different directions according to the driving of the driving plate, and the bending directions include upward, downward, inward, and outward. The mapping relationship can be as follows:
The drive plate 1' is retracted and the drive plate 3' is released: the internal catheter is bent upward;
The drive plate 3' is retracted and the drive plate 1' is released: the internal catheter is bent downward;
The drive plate 2' is retracted and the drive plate 4' is released: the internal catheter is bent outward (perpendicular to the paper surface and outward);
The drive plate 4' is retracted and the drive plate 2' is released: the internal catheter is bent inward (perpendicular to the paper surface and inward).

In FIG. 17, the driving plate 1' is connected to the transmission plate C, the driving plate 2' is connected to the transmission plate D, the driving plate 3' is connected to the transmission plate A, and the driving plate 4' is connected to the transmission plate B correspondingly. Due to the installation error, the first installation plate 4 needs to be rotated 180° together with the first catheter instrument 8 to the correct direction.

FIG. 18 is obtained after rotating FIG. 17, still maintaining the driving plate 1' is connected to the transmission plate C, the driving plate 2' is connected to the transmission plate D, the driving plate 3' is connected to the transmission plate A, and the driving plate 4' is connected to the transmission plate B correspondingly.

Different degrees of freedom may be in directions perpendicular to each other, for example, one degree of freedom may be in the up and down directions, and the other degree of freedom may be in the inside and outside directions.

At this time, the end of the external catheter can be bent in different directions according to the driving of the driving plate, and the bending directions include upward, downward, inward, and outward. The mapping relationship can be as follows:
The drive plate 3' is retracted and the drive plate 1' is released: the external catheter is bent upward;
The drive plate 1' is retracted and the drive plate 3' is released: the external catheter is bent downward;
The drive plate 4' is retracted and the drive plate 2' is released: the external catheter is bent outward (perpendicular to the paper surface and outward);
The drive plate 2' is retracted and the drive plate 4' is released: the external catheter is bent inward (perpendicular to the paper surface and inward).

The above is only one embodiment of the mapping relationship, and other modes that can realize this function can also be provided, which are not limited here. Referring to FIG. 20, another type of transmission plate and the retractable wire connection relationship of the catheter end are provided. a and b are located at the positions where a ray deviating from the upward direction at an angle of 45° intersects the circumference, and c and d are located at the positions where a ray deviating from the downward direction at an angle of 45° intersects the circumference. The difference between the scheme of FIG. 20 and the scheme of FIG. 19 is that a and b are used to collect the wire together (corresponding to transmission plates A and B), and d and c are used to release the wire together (corresponding to transmission plates B and D) to achieve the upward bending of the catheter end. Correspondingly, the three directions of downward, outward and inward are also controlled by the four transmission plates that retract and release the wires together. For example, BC retracts and AD releases correspondingly to outward bending. Further, the present application is not limited to the embodiments in FIG. 19 and FIG. 20 , and there may be a variety of connection relationships between the transmission plate and the retracting and releasing wire of the catheter end, so as to achieve bending of the catheter end in any direction, for example, the direction also includes an obliquely upward direction at any angle deviating from the upward direction.

Further, the mechanical arm includes a plurality of driving joints, the target motion direction includes the target feed motion direction of the catheter end, and the mapping relationship between the driving mechanism in the mechanical arm and the target motion direction of the catheter end configured according to the working direction includes:
Configuring a mapping relationship between the driving joints of the mechanical arm and the target feed motion direction according to the working direction.

Further, the target feed motion direction includes forward and backward, which respectively correspond to the forward and backward motion of the catheter end in the human body cavity. For example, when the working direction of the catheter robot is rightward, the corresponding target feed motion direction when the installation plate at the end of the mechanical arm moves to the right is forward; when the installation plate at the end of the mechanical arm moves to the left, the corresponding target feed motion direction is backward. When the working direction of the catheter robot is leftward, the corresponding target feed motion direction is forward when the installation plate at the end of the mechanical arm moves leftward, and the corresponding target feed motion direction is backward when the installation plate at the end of the mechanical arm moves rightward. Since the motion of the installation plate at the end of the mechanical arm to the left or right is affected by the motion of the driving joints in the mechanical arm, the driving joints in the mechanical arm have a corresponding mapping relationship with the target feed motion direction.

Further, the control method further includes:
configuring the mechanical arm with the external sheath instrument as an active mechanical arm, and configuring the mechanical arm with the internal tube instrument as a driven mechanical arm;
configuring the driven mechanical arm to move synchronously with the active mechanical arm. Further, the control method further includes:
setting a rotation direction and control parameters of the driving plate according to the configured mapping relationship; the control parameters include the motion speed. For example, in FIG. 18, the driving plate 3' is retracted and the driving plate 1' is released, and the corresponding external catheter bends upward, the driving disk 3' can be set to rotate clockwise for retracting the wire and counterclockwise for releasing the wire, the rotation speed can also be set separately according to whether the internal catheter or the external catheter is connected.

Further, controlling the driving mechanism to work according to the configured mapping relationship to realize the control of the catheter robot in the target motion direction. Wherein, including controlling the driving plate to achieve a change of the target bending motion direction of the catheter end, and controlling the driving joint to achieve the change of the target feed motion direction of the catheter end. On the other hand, the present application provides a catheter robot, including two mechanical arms and a processor, installation plates are respectively provided at the ends of the two mechanical arms, and the installation plates are used to install catheter instruments. The types of catheter instruments include internal tube instruments and external sheath instruments, and the processor is used to execute the above control method of the catheter robot.

On the other hand, the present application provides a readable storage medium having a computer program stored thereon, and when the computer program is executed by a processor, the steps of the above control method of catheter robot are implemented.

Referring to FIG. 14, in actual application, FIG. 14 is a structural schematic diagram of the hardware operating environment involved in the control method of catheter robot disclosed in the present application.

As shown in FIG. 14, the hardware operating environment may include: a processor 1001, such as a CPU, a communication bus 1002, a user interface 1003, a network interface 1004, and a memory 1005. The communication bus 1002 is used to realize the connection and communication between these components. The user interface 1003 may include a display screen (Display), an input unit such as a keyboard (Keyboard), and the optional user interface 1003 may also include a standard wired interface and a wireless interface. The network interface 1004 may optionally include a standard wired interface and a wireless interface (such as a WI-FI interface). The memory 1005 may be a high-speed RAM memory or a stable memory (non-volatile memory), such as a disk memory. The memory 1005 may also be a storage device independent of the processor 1001.

Those skilled in the art will appreciate that the hardware structure of the control method of catheter robot shown in FIG. 14 does not constitute a limitation on the device for operating the control method of catheter robot, and may include more or fewer components than shown in the figure, or a combination of certain components, or a different arrangement of components.

As shown in FIG. 14, the memory 1005 as a readable storage medium may include an operating system, a network communication module, a user interface module, and a computer program. The operating system is a management and control program that supports the operation of the network communication module, the user interface module, the computer program, and other programs or software; the network communication module is used to manage and control the network interface 1004; and the user interface module is used to manage and control the user interface 1003.

In the hardware structure shown in FIG. 14, the network interface 1004 is mainly used to connect to the backend server and perform data communication with the backend server; the user interface 1003 is mainly used to connect to the client (user end) and perform data communication with the client; the processor 1001 can call the computer program stored in the memory 1005 and perform the following steps:
automatically identifying the position and type of the catheter instrument when the catheter instrument is installed on the installation plate is detected;
determining the working direction of the catheter robot according to the position and type of the catheter instrument.

Further, after determining the working direction of the catheter robot,
identifying the installation direction of the catheter instrument;
configuring the mapping relationship between the driving mechanism in the mechanical arm and the target motion direction of the catheter end according to the working direction when the installation direction is consistent with the working direction.

Further, configuring the mapping relationship between the driving mechanism in the mechanical arm and the target motion direction of the catheter end according to the working direction, includes:
configuring the mapping relationship between the driving plate and the target bending motion direction according to the working direction and the physical connection correspondence between the driving plate and the transmission plate.

Further, configuring the mapping relationship between the driving mechanism in the mechanical arm and the target motion direction of the catheter end according to the working direction, includes:
configuring the mapping relationship between the driving joints and the target feed motion direction.

Further, configuring the mechanical arm with the external sheath instrument as the active mechanical arm, and configuring the mechanical arm with the internal tube instrument as the driven mechanical arm;
configuring the driven mechanical arm to move synchronously with the active mechanical arm.

Further, controlling the installation plate to rotate so that the installation direction is consistent with the working direction, when the installation direction is inconsistent with the working direction.

Further, after controlling the installation plate to rotate, configuring the mapping relationship between the driving mechanism in the mechanical arm and the target motion direction of the catheter end according to the working direction.

Further, after detecting that the catheter instrument is removed, controlling the installation plate to automatically rotates to the initial position.

Further, determining the working direction of the catheter robot according to the position and type of the catheter instrument further includes: if the position of the catheter instrument is the first position and the type of the first catheter instrument is the external sheath instrument, determining the working direction of the catheter robot as the first direction; if the position of the catheter instrument is the first position and the type of the first catheter instrument is the internal tube instrument, determining the working direction of the catheter robot as the second direction; if the position of the catheter instrument is the second position and the type of the second catheter instrument is the external sheath instrument, determining the working direction of the catheter robot as the second direction; if the position of the catheter instrument is the second position and the type of the second catheter instrument is the internal tube instrument, determining the working direction of the catheter robot as the first direction.

Further, identifying the installation direction of the catheter instrument includes:
If the identification device is identified by the first identifying device, the installation direction is the first direction;
If the identification device is identified by the second identifying device, the installation direction is the second direction. Further, setting the rotation direction and the control parameters of the driving plate according to the set mapping relationship; the control parameters include the motion speed.

Further, controlling the driving mechanism to work to realize the control of the catheter robot in the target motion direction according to the configured mapping relationship. Wherein, including controlling the driving plate to realize the change of the target bending motion direction of the catheter end, and controlling the driving joint to realize the change of the target feed motion direction of the catheter end.

In summary, the present application has the following beneficial effects: by utilizing the scheme of the present application, the type of the catheter instrument is automatically identified when the catheter instrument is installed on the installation plate, and the working direction of the catheter robot is determined according to the type of the catheter instrument, so that the working direction of the catheter robot can be automatically set without manually selecting the working direction or moving the robot to a new position, thereby improving work efficiency, reducing labor costs and further reducing the error rate.

Further, after determining the working direction of the catheter robot, outputting a prompt message to remind the user of the determined working direction, so that the user can further confirm whether the working direction needs to be changed. In this way, when the user has only installed one catheter instrument, the user can be reminded whether the installation is correct, without having to wait until all installations are completed to remind the user, thus saving the user's time. Further, after all the catheter instruments are installed, if it is determined that the types of all the catheter instruments are the same, an alarm message is issued to prompt the user to replace the catheter instrument, so that when the user installs the second catheter instrument, the user can be reminded whether it is installed correctly.

Further, the installation plate is equipped with the rotating device, which can automatically identify and correct the catheter instrument to the correct position when the user installs it incorrectly, without the need for manual operation by the user, further improving efficiency and enhancing user experience. Further, when it is detected that the catheter instrument has been removed, controlling installation plate to automatically rotate to the initial position. This reset operation can make it less likely to make mistakes during the next installation, and also reduce the complexity of the control program when detecting the direction.

Further, by utilizing the disclosed solution, when the installation direction is consistent with the working direction, configuring the mapping relationship between the driving mechanism in the mechanical arm and the target motion direction of the catheter end according to the working direction, thereby being able to adapt to a variety of working directions and angles of the catheter robot, expanding more application scenarios without the need for manual settings, and improving efficiency. Further, the method disclosed in the present application can also reset a new mapping relationship after detecting an installation error of the installation plate and performing rotation correction, so as to adapt to various rotation angles of the installation plate and the catheter instrument, expand more application scenarios, and eliminate the need for manual settings, thereby improving work efficiency.

Further, the present application provides another embodiment as follows.

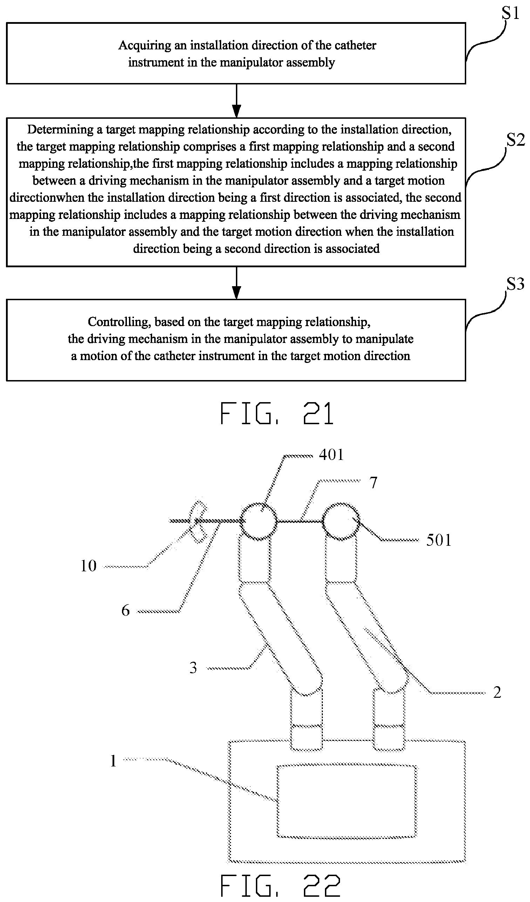

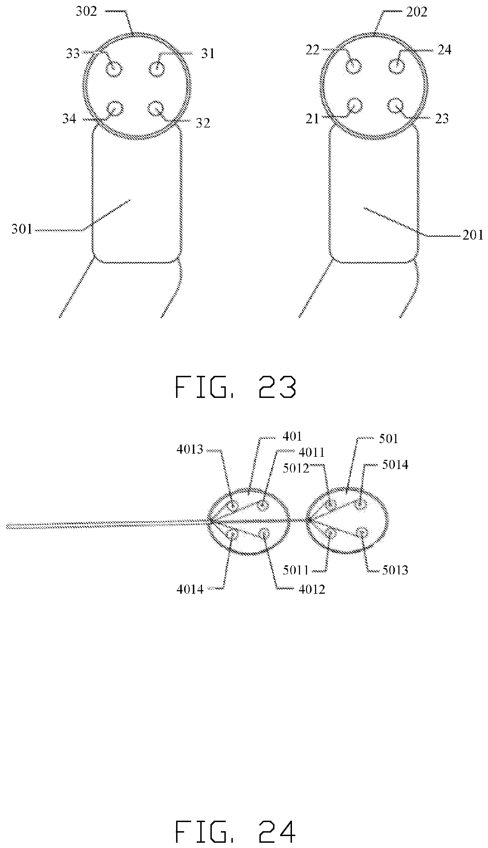

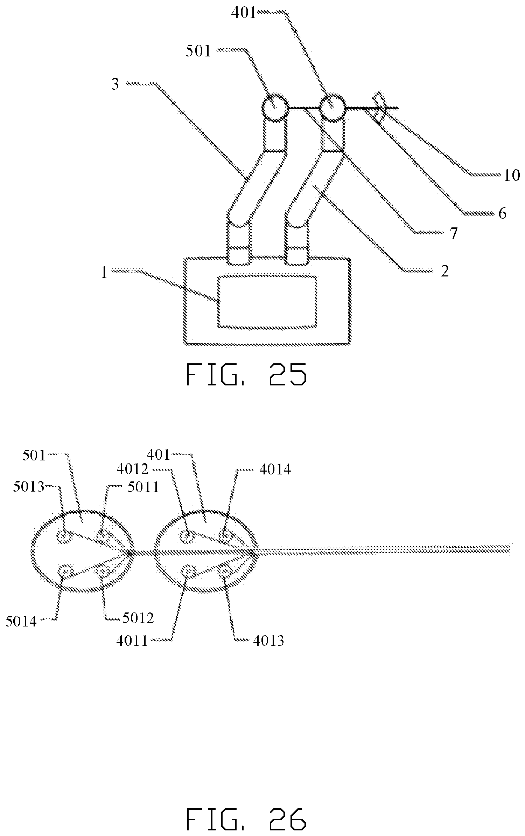

FIG. 21 is a flowchart of an embodiment of the present application of the control method of catheter robot. FIG. 22 is a structural schematic diagram of an embodiment of the present application of the catheter robot in a first direction. FIG. 23 is a structural schematic diagram of an embodiment of the present application of the mechanical arm and a manipulator assembly of the catheter robot. FIG. 24 is a structural schematic diagram of an embodiment of the present application of the catheter instrument when the catheter robot in the first direction. FIG. 25 is a structural schematic diagram of an embodiment of the present application of the catheter robot in a second direction. FIG. 26 is a structural schematic diagram of an embodiment of the present application of the catheter instrument when the catheter robot in the second direction.

As shown in FIGS. 21 to 26, the present disclosure provides a control method of catheter robot. The catheter robot includes a manipulator assembly, and the manipulator assembly is used to install and drive a catheter instrument. The control method includes:
Step S1: acquiring an installation direction of the catheter instrument in the manipulator assembly.
Step S2: determining a target mapping relationship according to the installation direction, the target mapping relationship comprises a first mapping relationship and a second mapping relationship.

Exemplary, the first mapping relationship includes a mapping relationship between a driving mechanism in the manipulator assembly and a target motion direction when the installation direction being a first direction is associated, the second mapping relationship includes a mapping relationship between the driving mechanism in the manipulator assembly and the target motion direction when the installation direction being a second direction is associated. That is, when the installation direction is the first direction, the first mapping relationship associated with the first direction is determined as the target mapping relationship from the first mapping relationship and the second mapping relationship; when the installation direction is the second direction, the second mapping relationship associated with the second direction is determined as the target mapping relationship from the first mapping relationship and the second mapping relationship.

Step S3: controlling, based on the target mapping relationship, the driving mechanism in the manipulator assembly to manipulate a motion of the catheter instrument in the target motion direction.

In some embodiments, as shown in FIGS. 22 to 25, the manipulator assembly includes a first manipulator assembly 2 and a second manipulator assembly 3, the catheter instrument includes an internal tube instrument 501 and an external sheath instrument 401, acquiring the installation direction of the catheter instrument in the manipulator assembly, includes:
determining the installation direction as the first direction when the internal tube instrument 501 installed to the first manipulator assembly 2 towards the first direction and the external sheath instrument 401 installed to the second manipulator assembly 3 towards the first direction are detected;
determining the installation direction as the second direction when the external sheath instrument 401 installed to the first manipulator assembly 2 towards the second direction and the internal tube instrument 501 installed to the second manipulator assembly 3 towards the second direction are detected;
the first direction is a direction of the first manipulator assembly 2 adjacent to a side of the second manipulator assembly 3, the second direction is a direction of the first manipulator assembly 2 away from the side of the second manipulator assembly 3. Exemplarily, the first direction and the second direction are usually opposite. In some embodiments, the first direction is the left direction and the second direction is the right direction. Of course, in other embodiments, the first direction may also be the right direction and the second direction may be the left direction.

In some embodiments, as shown in FIGS. 22 to 26, the first mapping relationship includes:
configuring a direction of the driving mechanism in the second manipulator assembly 3 manipulating a forward feed motion of the external sheath instrument 401, and a direction of the driving mechanism in the first manipulator assembly 2 manipulating a forward feed motion of the internal tube instrument 501 to be the same as the first direction. That is, in the first mapping relationship, for example, the motion of the external sheath instrument 401 and the internal tube instrument 501 toward the left direction is defined as the direction of forward motion, and the motion of the external sheath instrument 401 and the internal tube instrument 501 toward the right direction is defined as the direction of backward motion. It can be understood that since the feed motion includes forward feed motion and backward feed motion, the direction in which the driving mechanism in the second manipulator assembly 3 controls the external sheath instrument 401 to perform forward feed motion, and the direction in which the driving mechanism in the first manipulator assembly 2 controls the internal tube instrument 501 to perform forward feed motion are both the same as the first direction. In essence, it also includes the direction in which the driving mechanism in the second manipulator assembly 3 controls the external sheath instrument 401 to perform backward feed motion, and the direction in which the driving mechanism in the first manipulator assembly 2 controls the internal tube instrument 501 to perform backward feed motion are both opposite to the first direction. The above description is mainly for the purpose of simplicity.

Further, the first mapping relationship includes:
configuring the second manipulator assembly 3 as a main manipulator assembly, and the first manipulator assembly 2 as a vice manipulator assembly; configuring the vice manipulator assembly to move synchronously with the main manipulator assembly. That is, in the first mapping relationship, when the driving mechanism in the second manipulator assembly 3 manipulates the external sheath instrument 401 to move forward or backward, the driving mechanism in the first manipulator assembly 2 can, for example, follow the external sheath instrument 401 to manipulate the internal tube instrument 501 to move forward or backward at a ratio of 1:1. Because, in the process that external sheath instrument 401 and internal tube instrument 501 are inserted into an introducer 10, need to guarantee that external sheath instrument 401 and internal tube instrument 501 cannot move relative to each other, therefore, need to control external sheath instrument 401 and internal tube instrument 501 to carry out synchronous motion. Certainly, in the first manipulator assembly 2, driving mechanism for example can adopt other ratios to follow the external sheath instrument 401 to manipulate the internal tube instrument 501 to move forward or backward.

Alternatively, the vice manipulator assembly may also be configured to move independently, i.e., when actively controlling the vice manipulator assembly to move, the main manipulator assembly will not follow the vice manipulator assembly synchronous motion. For example, when actively controlling the internal tube instrument 501 to move, the external sheath instrument 401 will not follow the internal tube instrument 501 synchronous motions, because, after the external sheath instrument 401 and the internal tube instrument 501 insert in the introducer 10, it is necessary to control the internal tube instrument 501 motions separately to check the internal symptom situation, and now the external sheath instrument 401 follows the movement to affect normal inspection, and therefore, now, it is not necessary for the external sheath instrument 401 to follow the internal tube instrument 501 synchronous motions.

In some embodiments, as shown in FIG. 23, FIG. 25, and FIG. 26, the second mapping relationship includes:
configuring a direction of the driving mechanism in the first manipulator assembly 2 manipulating a forward feed motion of the external sheath instrument 401, and a direction of the driving mechanism in the second manipulator assembly 3 manipulating a forward feed motion of the internal tube instrument 501 to be the same as the second direction. That is, in the second mapping relationship, the external sheath instrument 401 and the internal tube instrument 501 moving toward the right direction is defined as the direction of forward motion, and the external sheath instrument 401 and the internal tube instrument 501 moving toward the left direction is defined as the direction of backward motion. It can be understood that the direction in which the driving mechanism in the first manipulator assembly 2 manipulates the external sheath instrument 401 to perform forward feed motion, and the direction in which the driving mechanism in the second manipulator assembly 3 manipulates the internal tube instrument 501 to perform forward feed motion are the same as the second direction, and the direction in which the driving mechanism in the first manipulator assembly 2 manipulates the external sheath instrument 401 to perform forward feed motion, and the direction in which the driving mechanism in the second manipulator assembly 3 manipulates the internal tube instrument 501 to perform forward feed motion are the same as the second direction, essentially also includes configuring the direction in which the driving mechanism in the first manipulator assembly 2 manipulates the external sheath instrument 401 to perform backward feed motion, and the direction in which the driving mechanism in the second manipulator assembly 3 manipulates the internal tube instrument 501 to perform backward feed motion is opposite to the second direction, and the aforementioned description is mainly for the purpose of simplicity.

Further, the second mapping relationship includes:
configuring the first manipulator assembly 2 as the main manipulator assembly, and the second manipulator assembly 3 as the vice manipulator assembly; configuring the vice manipulator assembly to move synchronously with the main manipulator assembly. That is, in the second mapping relationship, when the driving mechanism in the first manipulator assembly 2 manipulates the external sheath instrument 401 to move forward or backward, the driving mechanism in the second manipulator assembly 3 can, for example, adopt a 1:1 following motion of the external sheath instrument 401 to manipulate the internal tube instrument 501 to move forward or backward. Because, in the process that external sheath instrument 401 and internal tube instrument 501 are inserted into the introducer 10, need to guarantee that the external sheath instrument 401 and the internal tube instrument 501 cannot move relative to each other, therefore, need to control the external sheath instrument 401 and the internal tube instrument 501 to carry out synchronous motion. Certainly, in the second manipulator assembly 3, driving mechanism for example can adopt other ratios to follow the external sheath instrument 401 to manipulate the internal tube instrument 501 to move forward or backward.

Alternatively, the vice manipulator assembly may also be configured to move independently, i.e., when actively controlling the vice manipulator assembly to move, the main manipulator assembly will not follow the vice manipulator assembly synchronous motion. For example, when actively controlling the internal tube instrument 501 to move, the external sheath instrument 401 will not follow the internal tube instrument 501 synchronous motions, because, after the external sheath instrument 401 and the internal tube instrument 501 insert in the introducer 10, it is necessary to control the internal tube instrument 501 motions separately to check the internal symptom situation, and now the external sheath instrument 401 follows the movement to affect normal inspection, and therefore, now, it is not necessary for the external sheath instrument 401 to follow the internal tube instrument 501 synchronous motions.

In some embodiments, the manipulator assembly includes mechanical arms and installation plates installed on distal end of the mechanical arms, the installation plates are used to dock with the catheter instruments, and the installation plates may also be sometimes referred to as the manipulators. The mechanical arms may be generally configured to manipulate the catheter instruments to perform feed motions, and the installation plates may be generally configured to manipulate the catheter instruments to perform bending motions. The mechanical arm is provided with a plurality of actively driven joints, so that it can be configured to drive the feed motion of the catheter instrument forward or backward.

As shown in FIG. 23, the first manipulator assembly 2 includes a first mechanical arm 201 and a first installation plate 202, and the first installation plate 202 is arranged at the end of the first mechanical arm 201; the second manipulator assembly 3 includes a second mechanical arm 301 and a second installation plate 302, and the second installation plate 302 is arranged at the end of the second mechanical arm 301. One of the external sheath instrument 401 and the internal tube instrument 501 is installed on the first installation plate 202, and the other is installed on the second installation plate 302.

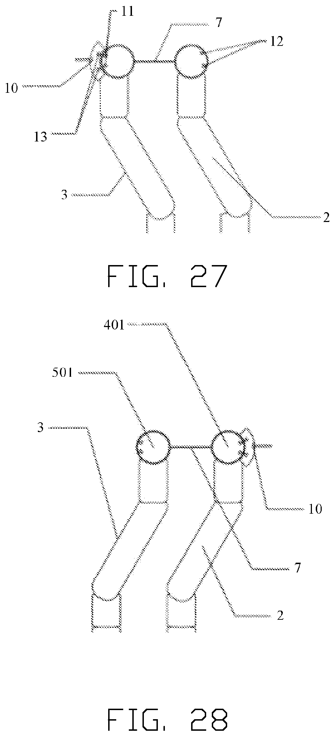

Further, referring to FIGS. 27 and 28, the first installation plate 202 is provided with a first positioning interface 12 on a side away from the second installation plate 302, the second installation plate 302 is provided with a second positioning interface 11 on a side away from the first installation plate 202, and the introducer 10 is provided with a third positioning interface 13 matching with the first positioning interface 12 and the second positioning interface 11. When the second positioning interface 11 and the third positioning interface 13 are matched, the robot end is matched with the introducer posture to ensure that the catheter enters the introducer in the correct position and direction, at this time, the manipulator assembly needs to work in the first mapping relationship. After the matching is completed, the internal and external catheters 6 are on the same straight line with the entrance of the introducer 10, and the working trajectory always moves on this straight line, the pre-matching process facilitates the operation. When the first positioning interface 12 cooperates with the third positioning interface 13, the manipulator assembly needs to work in the second mapping relationship. The manipulator assembly adopts a dual-interface design, and the catheter robot can adapt to the postures of the first mapping relationship and the second mapping relationship.

In some embodiments, a complementary structure may be formed between the first positioning interface 12 or the second positioning interface 11 and the third positioning interface 13, so as to realize mechanical positioning, for example. For example, the first positioning interface 12 and the second positioning interface 11 may be designed as notches, and accordingly, the third positioning interface 13 may be designed as a convex block complementary to the notch. Preferably, the notch can be a notch of an inverted cone structure, and the convex block is adaptively a convex block of a cone structure, and the opening size gradually shrinks in the direction of the convex block being inserted into the notch, so as to facilitate guidance and positioning during pairing. The inverted cone structure can be, for example, an inverted cone structure, and the vertebral structure corresponds to a cone structure.

In some embodiments, as shown in FIG. 23, the first manipulator assembly 2 includes the first installation plate 202 for connecting the catheter instrument, the second manipulator assembly 3 includes the second installation plate 302 for connecting the catheter instrument. The first mapping relationship includes:
configuring a first driving mechanism in the first installation plate 202 to drive the internal tube instrument 501 to manipulate the internal tube instrument 501 to move in a first bending direction, and configuring a second driving mechanism to drive the internal tube instrument 501 to manipulate the internal tube instrument 501 to move in a second bending direction;
configuring a third driving mechanism in the second installation plate 302 to drive the external sheath instrument 401 to manipulate the external sheath instrument 401 to move in the first bending direction, and configuring a fourth driving mechanism to drive the external sheath instrument 401 to manipulate the external sheath instrument 401 to move in the second bending direction;

Further, the second mapping relationship includes:
configuring the first driving mechanism in the first installation plate 202 to drive the external sheath instrument 401 to manipulate the external sheath instrument 401 to move in the first bending direction, and configuring the second driving mechanism to drive the external sheath instrument 401 to manipulate the external sheath instrument 401 to move in the second bending direction;
configuring the third driving mechanism in the second installation plate 302 to drive the internal tube instrument 501 to manipulate the internal tube instrument 501 to move in the first bending direction, and configuring the fourth driving mechanism to drive the internal tube instrument 501 to manipulate the internal tube instrument 501 to move in the second bending direction.

The first bending direction and the second bending direction can be motions in posture degrees of freedom. It should be noted that one bending direction includes motions in clockwise or counterclockwise directions in one posture degree of freedom, or one bending direction includes motions in upward or downward directions in one posture degree of freedom, or one bending direction includes motions in left or right directions in one posture degree of freedom.

Further, the internal tube instrument 501 is provided with an internal catheter 7, which controls the motion of the internal catheter 7 in the first bending direction and the second bending direction; the external sheath instrument 401 is provided with an external catheter 6, which controls the motion of the external catheter 6 in the first bending direction and the second bending direction. The internal tube 7 and the external tube 6 can be, for example, flexible tubes. Specifically, in the first mapping relationship, the first driving assembly drives the internal tube instrument 501 and controls the internal tube 7 to move in the first bending direction, and the second driving component drives the internal tube instrument 501 and controls the internal tube 7 to move in the second bending direction; the third driving assembly drives the external sheath instrument 401 and controls the external catheter 6 to move in the first bending direction, and the fourth driving assembly drives the external sheath instrument 401 and controls the external catheter 6 to move in the second bending direction. In the second mapping relationship, the first driving assembly drives the external sheath instrument 401 and controls the external catheter 6 to move in the first bending direction, and the second driving assembly drives the external sheath instrument 401 and controls the external catheter 6 to move in the second bending direction; the third driving assembly drives the internal tube instrument 501 and controls the internal catheter 7 to move in the first bending direction, and the fourth driving assembly drives the internal tube instrument 501 and controls the internal catheter 7 to move in the second bending direction. The first driving assembly and the third driving assembly, the second driving assembly and the fourth driving assembly are symmetrical about the same point, so as to facilitate the reversing installation of the catheter instrument.

In some embodiments, exemplarily as shown in FIG. 23, the first driving assembly includes a first driving plate 21 and a second driving plate 22, the second driving assembly includes a third driving plate 23 and a fourth driving plate 24; the third driving assembly includes a fifth driving plate 31 and a sixth driving plate 32, and the fourth driving assembly includes a seventh driving plate 33 and an eighth driving plate 34. The first driving plate 21 and the fifth driving plate 31, the second driving plate 22 and the sixth driving plate 32, the third driving plate 23 and the seventh driving plate 33, the fourth driving plate 24 and the eighth driving plate 34 are all symmetrical about the same point, that is, about the midpoint of the line connecting the first driving plate 21 and the fifth driving plate 31, the midpoint of the line connecting the second driving plate 22 and the sixth driving plate 32, the midpoint of the line connecting the third driving plate 23 and the seventh driving plate 33, or the midpoint of the line connecting the fourth driving plate 24 and the eighth driving plate 34.

As shown in FIGS. 24 and 26, in some embodiments, the first bending direction is the motion of the upper direction or the lower direction, and the second bending direction is the motion of the left direction or the right direction as an example for explanation. The internal tube instrument 501 is provided with a first upper transmission plate 5011, a first lower transmission plate 5012, a first left transmission plate 5013 and a first right transmission plate 5014, the first upper transmission plate 5011 and the first lower transmission plate 5012 are a group and are used to control the bending of the internal catheter 7 in the upper and lower directions, and the first left transmission plate 5013 and the first right transmission plate 5014 are a group and are used to control the bending of the internal catheter 7 in the left and right directions. The external sheath instrument 401 is provided with a second upper transmission plate 4011, a second lower transmission plate 4012, a second left transmission plate 4013 and a second right transmission plate 4014, the second upper transmission plate 4011 and the second lower transmission plate 4012 are a group and are used to control the bending of the external catheter 6 in the upper and lower directions, the second left transmission plate 4013 and the second right transmission plate 4014 are a group and are used to control the bending of the external catheter 6 in the left and right directions.

Each of the driving plates is used to drive the transmission plate matched therewith in the first mapping relationship or the second mapping relationship.

As shown in FIGS. 22 to 24, in the first mapping relationship, that is, when it is detected that the installation direction of the catheter instrument in the manipulator assembly is the first direction. The first driving plate 21 is used to drive the first upper transmission plate 5011, the second driving plate 22 is used to drive the first lower transmission plate 5012, the third driving plate 23 is used to drive the first left transmission plate 5013, and the fourth driving plate 24 is used to drive the first right transmission plate 5014; the fifth driving plate 31 is used to drive the second upper transmission plate 4011, the sixth driving plate 32 is used to drive the second lower transmission plate 4012, the seventh driving plate 33 is used to drive the second left transmission plate 4013, and the eighth driving plate 34 is used to drive the second right transmission plate 4014.

The following embodiments further illustrate the cooperation between the transmission plate and the driving wire, wherein the pull wire is the driving wire described above, including an upper pull wire, a lower pull wire, a left pull wire, a right pull wire, etc.

In one embodiment, the first driving plate 21 drives the first upper transmission plate 5011 to rotate and tighten the upper pull wire of the internal catheter 7, and the second driving plate 22 drives the first lower transmission plate 5012 to rotate and loosen the lower pull wire of the internal catheter 7, thereby controlling the internal catheter 7 to make a bending motion in the upper direction; when the first driving plate 21 and the second driving plate 22 are driven in reverse, the internal catheter 7 is controlled to make a bending motion in the lower direction. The third driving plate 23 drives the first left transmission plate 5013 to rotate and tighten the left pull wire of the internal catheter 7, and the fourth driving plate 24 drives the first right transmission plate 5014 to rotate and loosen the right pull wire of the internal catheter 7, thereby controlling the internal catheter 7 to perform bending motion in the left direction; when the third driving plate 23 and the fourth driving plate 24 are driven in reverse, the internal catheter 7 is controlled to perform bending motion in the right direction.

The fifth driving plate 31 drives the second upper transmission plate 4011 to rotate and tighten the upper pull wire of the external catheter 6, and the sixth driving plate 32 drives the second lower transmission plate 4012 to rotate and loosen the lower pull wire of the external catheter 6, thereby controlling the external catheter 6 to perform bending motion in the upper direction; when the fifth driving plate 31 and the sixth driving plate 32 are driven in reverse, the external catheter 6 is controlled to perform bending motion in the lower direction. The seventh driving plate 33 drives the second left transmission plate 4013 to rotate and tighten the left pull wire of the external catheter 6, and the eighth driving plate 34 drives the second right transmission plate 4014 to rotate and loosen the right pull wire of the external catheter 6, thereby controlling the external catheter 6 to make a bending motion in the left direction; when the seventh driving plate 33 and the eighth driving plate 34 are driven in reverse, the external catheter 6 is controlled to make a bending motion in the right direction.

As shown in FIGS. 23, 25 and 26, in the second mapping relationship, that is, when it is detected that the installation direction of the catheter instrument in the manipulator assembly is the second direction. The first driving plate 21 is used to drive the second upper transmission plate 4011, the second driving plate 22 is used to drive the second lower transmission plate 4012, the third driving plate 23 is used to drive the second left transmission plate 4013, and the fourth driving plate 24 is used to drive the second right transmission plate 4014; the fifth driving plate 31 is used to drive the first upper transmission plate 5011, the sixth driving plate 32 is used to drive the first lower transmission plate 5012, the seventh driving plate 33 is used to drive the first left transmission plate 5013, and the eighth driving plate 34 is used to drive the first right transmission plate 5014.

In one embodiment, the fifth driving plate 31 drives the first upper transmission plate 5011 to rotate and tighten the upper pull wire of the internal catheter 7, and the sixth driving plate 32 drives the first lower transmission plate 5012 to rotate and loosen the lower pull wire of the internal catheter 7, thereby controlling the internal catheter 7 to make a bending motion in the upper direction; when the fifth driving plate 31 and the sixth driving plate 32 are driven in reverse, the internal catheter 7 is controlled to make a bending motion in the lower direction. The seventh driving plate 33 drives the first left transmission plate 5013 to rotate and tighten the left pull wire of the internal catheter 7, and the eighth driving plate 34 drives the first right transmission plate 5014 to rotate and loosen the right pull wire of the internal catheter 7, thereby controlling the internal catheter 7 to make a bending motion in the left direction; when the seventh driving plate 33 and the eighth driving plate 34 are driven in reverse, the internal catheter 7 is controlled to make a bending motion in the right direction.

The first driving plate 21 drives the second upper transmission plate 4011 to rotate and tighten the upper pull wire of the external catheter 6, and the second driving plate 22 drives the second lower transmission plate 4012 to rotate and loosen the lower pull wire of the external catheter 6, thereby controlling the external catheter 6 to perform bending motion in the upper direction; when the first driving plate 21 and the second driving plate 22 are driven in reverse, the external catheter 6 is controlled to perform bending motion in the lower direction. The third driving plate 23 drives the second left transmission plate 4013 to rotate and tighten the left pull wire of the external catheter 6, and the fourth driving plate 24 drives the second right transmission plate 4014 to rotate and loosen the right pull wire of the external catheter 6, thereby controlling the external catheter 6 to perform bending motion in the left direction; when the third driving plate 23 and the fourth driving plate 24 are driven in reverse, the external catheter 6 is controlled to perform bending motion in the right direction.

In some embodiments, after determining the first mapping relationship is the target mapping relationship, the control method further includes:
detecting whether the first manipulator assembly 2 is installed with the internal tube instrument 501, and whether the second manipulator assembly 3 is installed with the external sheath instrument 401; when the first manipulator assembly 2 is not installed with the internal tube instrument 501, or the second manipulator assembly 3 is not installed with the external sheath instrument 401, control the catheter robot to raise an alarm. In actual operation, the possibility that the first manipulator assembly 2 and the second manipulator assembly 3 are both installed with the internal tube instrument 501 or the external sheath instrument 401 is not ruled out, in order to avoid erroneous operation, when it is detected that the first manipulator assembly 2 is not installed with the internal tube instrument 501, or the second manipulator assembly 3 is not installed with the external sheath instrument 401, the catheter robot is controlled to issue the alarm to remind the medical staff to reinstall it.

Similarly, after determining that the second mapping relationship is the target mapping relationship, the control method further includes:
detecting whether the first manipulator assembly is installed with the external sheath instrument, and whether the second manipulator assembly is installed with the internal tube instrument; when the first manipulator assembly is not installed with the external sheath instrument, or the second manipulator assembly is not installed with the internal tube instrument, control the catheter robot to raise an alarm. In actual operation, the possibility that the first manipulator assembly 2 and the second manipulator assembly 3 are both installed with the internal tube instrument 501 or the external sheath instrument 401 is not ruled out, in order to avoid erroneous operation, when it is detected that the first manipulator assembly is not installed with the external sheath instrument, or the second manipulator assembly is not installed with the internal tube instrument, the catheter robot is controlled to issue the alarm to remind the medical staff to reinstall it.

In some embodiments, an installation direction detection mechanism is provided between the manipulator assembly and the catheter instrument to obtain the installation direction of the catheter instrument in the manipulator assembly, including: detecting the installation direction of the catheter instrument in the manipulator assembly through the installation direction detection mechanism.

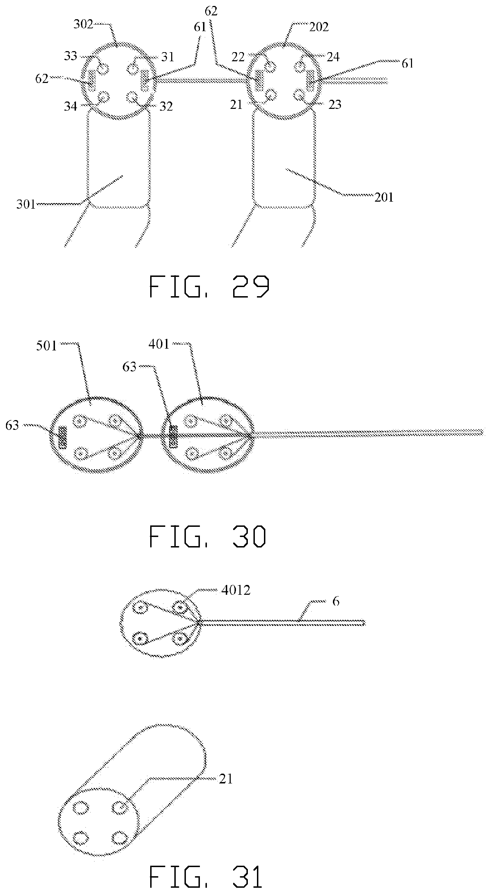

As shown in FIGS. 29 and 30, the installation direction detection mechanism includes: a first interface 61 and a second interface 62 installed on the manipulator assembly and an alignment interface 63 installed on the catheter instrument, and the alignment interface 63 selectively cooperates with the first interface 61 and the second interface 62. Specifically, the first installation plate 202 is provided with the first interface 61 and the second interface 62, the second installation plate 302 is provided with the first interface 61 and the second interface 62, the external sheath instrument 401 and the internal tube instrument 501 are both provided with the alignment interface 63, the alignment interface 63 is located at one end of the external sheath instrument 401 away from the extension direction of the external catheter 6, and the alignment interface 63 is located at one end of the internal tube instrument 501 away from the extension direction of the internal catheter 7. As shown in FIGS. 29 and 30, when it is detected that the alignment interface 63 of the internal tube instrument 501 cooperates with the first interface 61 of the first installation plate 202, and it is detected that the alignment interface 63 of the external sheath instrument 401 cooperates with the first interface 61 of the second installation plate 302, it is usually expected that the first mapping relationship is the target mapping relationship. When it is detected that the alignment interface 63 of the internal tube instrument 501 matches the second interface 62 of the second installation plate 302, and it is detected that the alignment interface 63 of the external sheath instrument 401 matches the second interface 62 of the first installation plate 202, it is usually expected that the second mapping relationship is the target mapping relationship.

In some embodiments, the alignment interface 63 may also be provided at one end of the catheter instrument toward the extension direction of the catheter, specifically, the alignment interface 63 is located at one end of the external sheath instrument 401 toward the extension direction of the external catheter 6, and the alignment interface 63 is located at one end of the internal tube instrument 501 toward the extension direction of the internal catheter 7. When it is detected that the alignment interface 63 is matched with the second interface 62, it can be determined that the catheter is facing the first direction of the catheter robot and the catheter instrument is in the first installation direction; when it is detected that the alignment interface 63 is matched with the first interface 61, it can be determined that the catheter is facing the second direction of the catheter robot and the catheter instrument is in the second installation direction. Particularly, when detecting that the alignment interface 63 of the internal tube instrument 501 cooperates with the second interface 62 of the first installation plate 202, and detecting that the alignment interface 63 of the external sheath instrument 401 cooperates with the second interface 62 of the second installation plate 302, it is usually desired to realize that the first mapping relationship is the target mapping relationship. When detecting that the alignment interface 63 of the internal tube instrument 501 cooperates with the first interface 61 of the second installation plate 302, and detecting that the alignment interface 63 of the external sheath instrument 401 cooperates with the first interface 61 of the first installation plate 202, it is usually expected that the second mapping relationship is the target mapping relationship.

Of course, in some embodiments, the first interface 61 and the second interface 62 may be installed on the catheter instrument, and the alignment interface 63 may be installed on the manipulator assembly, that is, the external sheath instrument 401 is provided with the first interface 61 and the second interface 62, the internal tube instrument 501 is provided with the first interface 61 and the second interface 62, and the first installation plate 202 and the second installation plate 302 are both provided with the alignment interface 63. It is only necessary to be able to identify the installation direction of the catheter instrument on the manipulator assembly.

In another embodiment, the installation direction detection mechanism includes: a first magnetic receiver and a second magnetic receiver installed on the catheter instrument and a magnetic member installed on the catheter instrument, and the magnetic member selectively cooperates with the first magnetic receiver and the second magnetic receiver. The magnetic member selectively cooperates with the first magnetic receiver and the second magnetic receiver to identify the installation direction of the catheter instrument in the manipulator assembly. That is, the first magnetic receiver replaces the first interface 61, the second magnetic receiver replaces the second interface 62, and the magnetic member replaces the alignment interface 63.

Further, the external sheath instrument 401 and the internal tube instrument 501 are both provided with electronic identification modules corresponding to the external sheath instrument 401 and the internal tube instrument 501, and the electronic identification modules are electrically connected to the alignment interface 63. The catheter robot is electrically connected to the first interface 61 or the second interface 62 through the alignment interface 63, thereby identifying the installation direction of the catheter instrument and whether the catheter instrument is the external sheath instrument 401 or the internal tube instrument 501.

In one embodiment, the catheter robot is provided with an installation direction input module, and obtaining the installation direction of the catheter instrument in the manipulator assembly includes: obtaining the installation direction of the catheter instrument in the manipulator assembly through the installation direction input module. The installation direction input module can be a button, a touch screen, a voice or other module, so that the installation direction can be manually input to the catheter robot to realize the confirmation of the installation direction.

Of course, in one embodiment, an image sensor can also be installed on the catheter robot, with the external sheath instrument 401 and the internal tube instrument 501 obtained by the image sensor in the installation direction of the manipulator assembly, to update the mapping relationship below. A mark can be set on the external sheath instrument 401 and the internal tube instrument 501 that need to be identified, so as to be convenient to determine.

The present application also provides a catheter robot, which is controlled by the control method as described above, and the catheter robot includes:
a manipulator assembly, the manipulator assembly includes mechanical arms and installation plates, the mechanical arm is provided with a plurality of joints for driving the catheter instrument to move forward or backward. As shown in FIG. 23, the first manipulator assembly 2 includes a first mechanical arm 201 and a first installation plate 202, and the first installation plate 202 is disposed at the end of the first mechanical arm 201; the second manipulator assembly 3 includes a second mechanical arm 301 and a second installation plate 302, and the second installation plate 302 is disposed at the end of the second mechanical arm 301;
and a control apparatus, the control apparatus is coupled with the manipulator assembly and configured to: acquire an installation direction of the catheter instrument in the manipulator assembly; determine a target mapping relationship from one of a first mapping relationship and a second mapping relationship according to the installation direction, the first mapping relationship includes a mapping relationship between a driving mechanism in the manipulator assembly and a target motion direction when the installation direction being a first direction is associated, the second mapping relationship includes a mapping relationship between the driving mechanism in the manipulator assembly and the target motion direction when the installation direction being a second direction is associated; the control apparatus can be a control chip, i.e. a processor.
control, based on the target mapping relationship, the driving mechanism in the manipulator assembly to manipulate a motion of the catheter instrument in the target motion direction.

Further, the catheter instrument includes the external sheath instrument 401 and the internal tube instrument 501, the external sheath instrument 401 is provided with the external catheter 6, and the external sheath instrument 401 controls the motion of the external catheter 6 in the first bending direction and the second bending direction. The internal tube instrument 501 is provided with an internal catheter 7, and the internal tube instrument 501 controls the motion of the internal catheter 7 in the first bending direction and the second bending direction.

Further, the first installation plate 202 is provided with a first driving assembly and a second driving assembly, and the second installation plate 302 is provided with a third driving assembly and a fourth driving assembly, the first driving assembly and the third driving assembly, and the second driving assembly and the fourth driving assembly are symmetrical about the same point, so as to facilitate the reversing installation of the catheter instrument. In the first mapping relationship, the first driving assembly in the first installation plate 202 drives the internal tube instrument 501 and manipulates the internal tube instrument 501 to move in the first bending direction, and the second driving assembly drives the internal tube instrument 501 and manipulates the internal tube instrument 501 to move in the second bending direction; the third driving assembly in the second installation plate 302 drives the external sheath instrument 401 and manipulates the external sheath instrument 401 to move in the first bending direction, and the fourth driving assembly drives the external sheath instrument 401 and manipulates the external sheath instrument 401 to move in the second bending direction. In the second mapping relationship, the first driving assembly drives the external sheath instrument 401 and manipulates the external sheath instrument 401 to move in the first bending direction, and the second driving assembly in the first installation plate 202 drives the external sheath instrument 401 and manipulates the external sheath instrument 401 to move in the second bending direction; the third driving assembly in the second installation plate 302 drives the internal tube instrument 501 and manipulates the internal tube instrument 501 to move in the first bending direction, and the fourth driving assembly drives the internal tube instrument 501 and manipulates the internal tube instrument 501 to move in the second bending direction.

In some embodiments, exemplarily as shown in FIG. 23, the first driving assembly includes the first driving plate 21 and the second driving plate 22, the second driving assembly includes the third driving plate 23 and the fourth driving plate 24; the third driving assembly includes a fifth driving plate 31 and a sixth driving plate 32, and the fourth driving assembly includes a seventh driving plate 33 and an eighth driving plate 34. The first driving plate 21 and the fifth driving plate 31, the second driving plate 22 and the sixth driving plate 32, the third driving plate 23 and the seventh driving plate 33, the fourth driving plate 24 and the eighth driving plate 34 are all symmetrical about the same point, that is, about the midpoint of the line connecting the first driving plate 21 and the fifth driving plate 31, the midpoint of the line connecting the second driving plate 22 and the sixth driving plate 32, the midpoint of the line connecting the third driving plate 23 and the seventh driving plate 33, or the midpoint of the line connecting the fourth driving plate 24 and the eighth driving plate 34.

As shown in FIGS. 24 and 26, in some embodiments, the first bending direction is the motion of the upper direction or the lower direction, and the second bending direction is the motion of the left direction or the right direction as an example for explanation. The internal tube instrument 501 is provided with a first upper transmission plate 5011, a first lower transmission plate 5012, a first left transmission plate 5013 and a first right transmission plate 5014, the first upper transmission plate 5011 and the first lower transmission plate 5012 are a group and are used to control the bending of the internal catheter 7 in the upper and lower directions, and the first left transmission plate 5013 and the first right transmission plate 5014 are a group and are used to control the bending of the internal catheter 7 in the left and right directions. The external sheath instrument 401 is provided with a second upper transmission plate 4011, a second lower transmission plate 4012, a second left transmission plate 4013 and a second right transmission plate 4014, the second upper transmission plate 4011 and the second lower transmission plate 4012 are a group and are used to control the bending of the external catheter 6 in the upper and lower directions, the second left transmission plate 4013 and the second right transmission plate 4014 are a group and are used to control the bending of the external catheter 6 in the left and right directions.

Each of the driving plates is used to drive the transmission plate matched therewith in the first mapping relationship or the second mapping relationship.

As shown in FIGS. 22 to 24, in the first mapping relationship, that is, when it is detected that the installation direction of the catheter instrument in the manipulator assembly is the first direction. The first driving plate 21 is used to drive the first upper transmission plate 5011, the second driving plate 22 is used to drive the first lower transmission plate 5012, the third driving plate 23 is used to drive the first left transmission plate 5013, and the fourth driving plate 24 is used to drive the first right transmission plate 5014; the fifth driving plate 31 is used to drive the second upper transmission plate 4011, the sixth driving plate 32 is used to drive the second lower transmission plate 4012, the seventh driving plate 33 is used to drive the second left transmission plate 4013, and the eighth driving plate 34 is used to drive the second right transmission plate 4014.

In one embodiment, the first driving plate 21 drives the first upper transmission plate 5011 to rotate and tighten the upper pull wire of the internal catheter 7, and the second driving plate 22 drives the first lower transmission plate 5012 to rotate and loosen the lower pull wire of the internal catheter 7, thereby controlling the internal catheter 7 to make a bending motion in the upper direction; when the first driving plate 21 and the second driving plate 22 are driven in reverse, the internal catheter 7 is controlled to make a bending motion in the lower direction. The third driving plate 23 drives the first left transmission plate 5013 to rotate and tighten the left pull wire of the internal catheter 7, and the fourth driving plate 24 drives the first right transmission plate 5014 to rotate and loosen the right pull wire of the internal catheter 7, thereby controlling the internal catheter 7 to perform bending motion in the left direction; when the third driving plate 23 and the fourth driving plate 24 are driven in reverse, the internal catheter 7 is controlled to perform bending motion in the right direction.

The fifth driving plate 31 drives the second upper transmission plate 4011 to rotate and tighten the upper pull wire of the external catheter 6, and the sixth driving plate 32 drives the second lower transmission plate 4012 to rotate and loosen the lower pull wire of the external catheter 6, thereby controlling the external catheter 6 to perform bending motion in the upper direction; when the fifth driving plate 31 and the sixth driving plate 32 are driven in reverse, the external catheter 6 is controlled to perform bending motion in the lower direction. The seventh driving plate 33 drives the second left transmission plate 4013 to rotate and tighten the left pull wire of the external catheter 6, and the eighth driving plate 34 drives the second right transmission plate 4014 to rotate and loosen the right pull wire of the external catheter 6, thereby controlling the external catheter 6 to make a bending motion in the left direction; when the seventh driving plate 33 and the eighth driving plate 34 are driven in reverse, the external catheter 6 is controlled to make a bending motion in the right direction.

As shown in FIGS. 23, 25 and 26, in the second mapping relationship, that is, when it is detected that the installation direction of the catheter instrument in the manipulator assembly is the second direction. The first driving plate 21 is used to drive the second upper transmission plate 4011, the second driving plate 22 is used to drive the second lower transmission plate 4012, the third driving plate 23 is used to drive the second left transmission plate 4013, and the fourth driving plate 24 is used to drive the second right transmission plate 4014; the fifth driving plate 31 is used to drive the first upper transmission plate 5011, the sixth driving plate 32 is used to drive the first lower transmission plate 5012, the seventh driving plate 33 is used to drive the first left transmission plate 5013, and the eighth driving plate 34 is used to drive the first right transmission plate 5014.

In one embodiment, the fifth driving plate 31 drives the first upper transmission plate 5011 to rotate and tighten the upper pull wire of the internal catheter 7, and the sixth driving plate 32 drives the first lower transmission plate 5012 to rotate and loosen the lower pull wire of the internal catheter 7, thereby controlling the internal catheter 7 to make a bending motion in the upper direction; when the fifth driving plate 31 and the sixth driving plate 32 are driven in reverse, the internal catheter 7 is controlled to make a bending motion in the lower direction. The seventh driving plate 33 drives the first left transmission plate 5013 to rotate and tighten the left pull wire of the internal catheter 7, and the eighth driving plate 34 drives the first right transmission plate 5014 to rotate and loosen the right pull wire of the internal catheter 7, thereby controlling the internal catheter 7 to make a bending motion in the left direction; when the seventh driving plate 33 and the eighth driving plate 34 are driven in reverse, the internal catheter 7 is controlled to make a bending motion in the right direction.

The first driving plate 21 drives the second upper transmission plate 4011 to rotate and tighten the upper pull wire of the external catheter 6, and the second driving plate 22 drives the second lower transmission plate 4012 to rotate and loosen the lower pull wire of the external catheter 6, thereby controlling the external catheter 6 to perform bending motion in the upper direction; when the first driving plate 21 and the second driving plate 22 are driven in reverse, the external catheter 6 is controlled to perform bending motion in the lower direction. The third driving plate 23 drives the second left transmission plate 4013 to rotate and tighten the left pull wire of the external catheter 6, and the fourth driving plate 24 drives the second right transmission plate 4014 to rotate and loosen the right pull wire of the external catheter 6, thereby controlling the external catheter 6 to perform bending motion in the left direction; when the third driving plate 23 and the fourth driving plate 24 are driven in reverse, the external catheter 6 is controlled to perform bending motion in the right direction.

In one embodiment, the catheter robot is provided with a type detection module, which is used to detect the type of the catheter instrument installed on the manipulator assembly, that is, to detect whether the internal tube instrument 501 or the external sheath instrument 401 is installed on the manipulator assembly.

In one embodiment, before determining the first mapping relationship as the target mapping relationship, detecting whether the first manipulator assembly 2 is installed with the internal tube instrument 501, and whether the second manipulator assembly 3 is installed with the external sheath instrument 401 through the type detection module; when the first manipulator assembly 2 is not installed with the internal tube instrument 501, or the second manipulator assembly 3 is not installed with the external sheath instrument 401, control the catheter robot to raise an alarm. In actual operation, the possibility that the first manipulator assembly 2 and the second manipulator assembly 3 are both installed with the internal tube instrument 501 or the external sheath instrument 401 is not ruled out, in order to avoid erroneous operation, when it is detected that the first manipulator assembly 2 is not installed with the internal tube instrument 501, or the second manipulator assembly 3 is not installed with the external sheath instrument 401, the catheter robot is controlled to issue the alarm to remind the medical staff to reinstall it.

Similarly, before determining the second mapping relationship as the target mapping relationship, detecting whether the first manipulator assembly is installed with the external sheath instrument, and whether the second manipulator assembly is installed with the internal tube instrument through the type detection module; when the first manipulator assembly is not installed with the external sheath instrument, or the second manipulator assembly is not installed with the internal tube instrument, control the catheter robot to raise an alarm. In actual operation, the possibility that the first manipulator assembly 2 and the second manipulator assembly 3 are both installed with the internal tube instrument 501 or the external sheath instrument 401 is not ruled out, in order to avoid erroneous operation, when it is detected that the first manipulator assembly is not installed with the external sheath instrument, or the second manipulator assembly is not installed with the internal tube instrument, the catheter robot is controlled to issue the alarm to remind the medical staff to reinstall it.

In one embodiment, the installation direction detection mechanism is provided between the manipulator assembly and the catheter instrument to detect the installation direction of the catheter instrument in the manipulator assembly.

As shown in FIGS. 29 and 30, the installation direction detection mechanism includes: a first interface 61 and a second interface 62 installed on the manipulator assembly and an alignment interface 63 installed on the catheter instrument, and the alignment interface 63 selectively cooperates with the first interface 61 and the second interface 62. Specifically, the first installation plate 202 is provided with the first interface 61 and the second interface 62, the second installation plate 302 is provided with the first interface 61 and the second interface 62, the external sheath instrument 401 and the internal tube instrument 501 are both provided with the alignment interface 63, the alignment interface 63 is located at one end of the external sheath instrument 401 away from the extension direction of the external catheter 6, and the alignment interface 63 is located at one end of the internal tube instrument 501 away from the extension direction of the internal catheter 7. As shown in FIGS. 29 and 30, when it is detected that the alignment interface 63 of the internal tube instrument 501 cooperates with the first interface 61 of the first installation plate 202, and it is detected that the alignment interface 63 of the external sheath instrument 401 cooperates with the first interface 61 of the second installation plate 302, it is usually expected that the first mapping relationship is the target mapping relationship. When it is detected that the alignment interface 63 of the internal tube instrument 501 matches the second interface 62 of the second installation plate 302, and it is detected that the alignment interface 63 of the external sheath instrument 401 matches the second interface 62 of the first installation plate 202, it is usually expected that the second mapping relationship is the target mapping relationship.

In some embodiments, the alignment interface 63 may also be provided at one end of the catheter instrument toward the extension direction of the catheter, specifically, the alignment interface 63 is located at one end of the external sheath instrument 401 toward the extension direction of the external catheter 6, and the alignment interface 63 is located at one end of the internal tube instrument 501 toward the extension direction of the internal catheter 7. When it is detected that the alignment interface 63 is matched with the second interface 62, it can be determined that the catheter is facing the first direction of the catheter robot and the catheter instrument is in the first installation direction; when it is detected that the alignment interface 63 is matched with the first interface 61, it can be determined that the catheter is facing the second direction of the catheter robot and the catheter instrument is in the second installation direction. Particularly, when detecting that the alignment interface 63 of the internal tube instrument 501 cooperates with the second interface 62 of the first installation plate 202, and detecting that the alignment interface 63 of the external sheath instrument 401 cooperates with the second interface 62 of the second installation plate 302, it is usually desired to realize that the first mapping relationship is the target mapping relationship. When detecting that the alignment interface 63 of the internal tube instrument 501 cooperates with the first interface 61 of the second installation plate 302, and detecting that the alignment interface 63 of the external sheath instrument 401 cooperates with the first interface 61 of the first installation plate 202, it is usually expected that the second mapping relationship is the target mapping relationship.

Of course, in some embodiments, the first interface 61 and the second interface 62 may be installed on the catheter instrument, and the alignment interface 63 may be installed on the manipulator assembly, that is, the external sheath instrument 401 is provided with the first interface 61 and the second interface 62, the internal tube instrument 501 is provided with the first interface 61 and the second interface 62, and the first installation plate 202 and the second installation plate 302 are both provided with the alignment interface 63. It is only necessary to be able to identify the installation direction of the catheter instrument on the manipulator assembly.

In another embodiment, the installation direction detection mechanism includes: a first magnetic receiver and a second magnetic receiver installed on the catheter instrument and a magnetic member installed on the catheter instrument, and the magnetic member selectively cooperates with the first magnetic receiver and the second magnetic receiver. The magnetic member selectively cooperates with the first magnetic receiver and the second magnetic receiver to identify the installation direction of the catheter instrument in the manipulator assembly. That is, the first magnetic receiver replaces the first interface 61, the second magnetic receiver replaces the second interface 62, and the magnetic member replaces the alignment interface 63.

Further, the external sheath instrument 401 and the internal tube instrument 501 are both provided with electronic identification modules corresponding to the external sheath instrument 401 and the internal tube instrument 501, and the electronic identification modules are electrically connected to the alignment interface 63. The catheter robot is electrically connected to the first interface 61 or the second interface 62 through the alignment interface 63, thereby identifying the installation direction of the catheter instrument and whether the catheter instrument is the external sheath instrument 401 or the internal tube instrument 501.

In one embodiment, the catheter robot is provided with the installation direction input module, and obtaining the installation direction of the catheter instrument in the manipulator assembly includes: obtaining the installation direction of the catheter instrument in the manipulator assembly through the installation direction input module. The installation direction input module can be a button, a touch screen, a voice or other module, so that the installation direction can be manually input to the catheter robot to realize the confirmation of the installation direction.

Of course, in one embodiment, an image sensor can also be installed on the catheter robot, with the external sheath instrument 401 and the internal tube instrument 501 obtained by the image sensor in the installation direction of the manipulator assembly, to update the mapping relationship below. A mark can be set on the external sheath instrument 401 and the internal tube instrument 501 that need to be identified, so as to be convenient to determine.

The present application also provides a computer-readable storage medium, storing a computer program, and the computer program is configured to be loaded and executed by a processor to implement the steps of the control method described above.

The present application also provides a control apparatus, including:
a memory, storing a computer program;
and a processor, loading and executing the computer program;
wherein, the computer program is configured to be loaded and executed by the processor to implement the steps of the control method as described above.

Wherein, the memory may include a high-speed RAM memory, and may also include a non-volatile memory, such as at least one disk memory.

Wherein, the processor may be a central processing unit (CPU), or an application-specific integrated circuit (ASIC), or one or more integrated circuits configured to implement the embodiments of the present application, or a graphics processing unit (GPU). The one or more processors included in the control device may be processors of the same type, such as one or more CPUs, or one or more GPUs; or may be processors of different types, such as one or more CPUs and one or more GPUs.

In some embodiments, the control apparatus may be integrated into the catheter robot setting. The control apparatus may also be independent of the catheter robot setting. The control apparatus may also be deployed in the cloud.

The technical features of the above-mentioned embodiments can be combined arbitrarily. In order to make the description concise, all possible combinations of the technical features in the above-mentioned embodiments are not described. However, as long as there is no contradiction in the combination of these technical features, they should be considered to be within the scope of the description of this specification.

The technical features of the above-mentioned embodiments and the combination of any technical features are universal. They are not only applicable to single-hole surgical robots, but also to multi-hole surgical robots. Moreover, they will neither affect nor limit the use in robotic arms with different configurations.

Further, the present disclosure provides another embodiment below.

As shown in FIGS. 2, 22, and 25, the catheter robot of the embodiment of the present disclosure includes a trolley and a manipulator assembly installed on the trolley, a catheter instrument installed on the manipulator assembly and driven to move by the manipulator assembly, and a control apparatus coupled to the manipulator assembly.

The control apparatus may be a central processing unit (CPU), or an application specific integrated circuit (ASIC), or one or more integrated circuits configured to implement the embodiments of the present application, or a graphics processing unit (GPU). The one or more processors included in the control apparatus may be processors of the same type, such as one or more CPUs, or one or more GPUs; or may be processors of different types, such as one or more CPUs and one or more GPUs. In some embodiments, the control apparatus may be integrated into the catheter robot setting. The control device may also be independent of the catheter robot setting. The control apparatus may also be deployed in the cloud.

As shown in FIGS. 22 to 25, the manipulator assembly includes a first manipulator assembly 2 and a second manipulator assembly 3, the catheter instrument includes an internal tube instrument 501 and an external sheath instrument 401. The internal tube instrument 501 can be selectively assembled on the first manipulator assembly 3 or the second manipulator assembly 2. When the internal tube instrument 501 is installed on the first manipulator assembly 3, the external sheath instrument 401 is installed on the second manipulator assembly 2. When the internal tube instrument 501 is installed on the second manipulator assembly 2, the external sheath instrument 401 is installed on the first manipulator assembly 3.

The catheter instrument is used for removably connecting with the manipulator assembly, a catheter is connected in the catheter instrument, and the catheter is driven by the catheter instrument to perform bending motion. For example, the catheter of the internal tube instrument 501 can be defined as the internal catheter 7, and the catheter of the external sheath instrument 401 can be defined as the external catheter 6.

When in use, the internal tube instrument 501 and the external sheath instrument 401 are oriented in the same direction, and the internal catheter 7 first passes through the catheter instrument of the external sheath instrument 4 and then passes through the external catheter 6.

As shown in FIGS. 22 and 23, the first manipulator assembly 3 includes a first mechanical arm 301 and a first installation plate 302, the second manipulator assembly 2 includes a second mechanical arm 201 and a second installation plate 202. The internal tube instrument 501 and the external sheath instrument 401 can be installed on the first installation plate 302 and the second installation plate 202, respectively, or can also be installed on the second installation plate 202 and the first installation plate 302, respectively. The motion of the first mechanical arm 301 and the second mechanical arm 201 controls the feed motion of the internal catheter end and the external catheter end, for example, the internal catheter end moves forward or backward, and the first installation plate 302 and the second installation plate 202 drive the catheter instrument to work to control the bending motion of the internal catheter end and the external catheter end, for example, the internal catheter end bends to the left or to the right.

In some embodiments, the present disclosure provides a zeroing method for a catheter instrument after it is installed on the installation plate, which is mainly used to adjust the catheter end in the catheter instrument to be in a straight state. At this time, the catheter end is in a zero position state. Since the first installation plate and the second installation plate have substantially the same structure, the internal tube instrument and the external sheath instrument also have substantially the same structure, and thus, the installation and zeroing of any catheter instrument and any installation plate can be taken as an example for description.

Exemplarily, as shown in FIG. 31, the structural components related to the embodiment of the present disclosure are described by taking the first installation plate 202 and the external sheath instrument 401 as examples. The first installation plate 202 includes a driving plate 21 and a sensor (not shown) for detecting the position of the driving plate 21; the external sheath instrument 401 includes a catheter instrument and a catheter 6 connected to the catheter instrument and driven by the catheter instrument. The catheter instrument includes a transmission plate 4012, and the transmission plate 4012 is connected to and drives the end of the catheter 6 to perform bending motion such as the end instrument. The catheter instrument and the installation plate can be removably connected through the transmission plate 4012 and the driving plate 21.

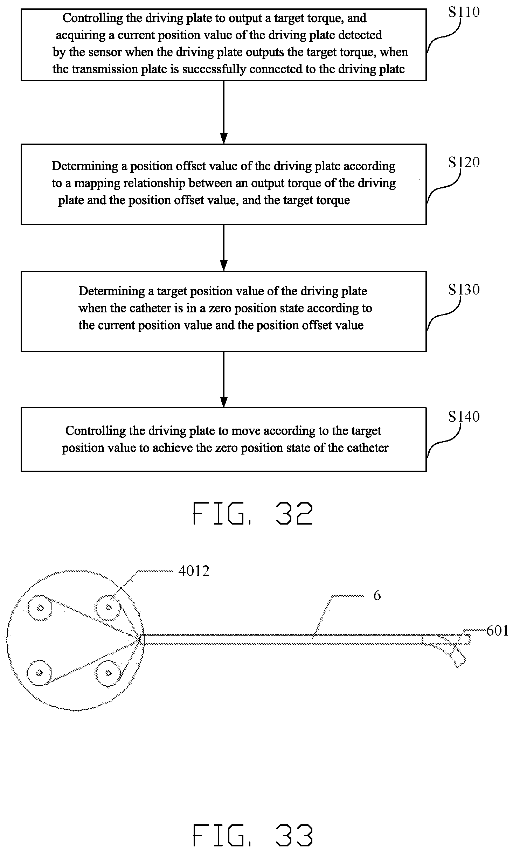

As shown in FIG. 32, in an embodiment of the present disclosure, the control apparatus is configured to execute:
Step S110: controlling the driving plate to output a target torque, and acquiring a current position value of the driving plate detected by the sensor when the driving plate outputs the target torque, when the transmission plate is successfully connected to the driving plate;

In some embodiments, when the transmission plate 4012 is successfully connected and matched with the driving plate 21, the driving plate 21 is controlled to output a target torque along the direction of the pull wire bent at the end of the pulling catheter, and the current position value of the driving plate 21 when outputting the target torque detected by the sensor is obtained, the current position value is a stable position value detected by the sensor. The sensor may be a motor encoder or other sensor capable of detecting the current position value, which is not limited in the present disclosure. The following example illustrates that, for example, controlling the target torque value output by the driving plate 21 is 10N, and the current position value detected by the sensor is 65.3mm.

As an optional embodiment, controlling the driving plate to output the target torque includes: determining a target rotation direction in which the drive plate pulls the end of the catheter to bend through the transmission plate according to the mapping relationship between the rotation direction of the driving plate and a bend direction of the end of the catheter; controlling the driving plate to output the target torque in the target rotation direction. In some embodiments, the target rotation direction of the driving plate may refer to the direction of the pull wire of the catheter end to bend through the transmission plate.

In some embodiments, as shown in FIGS. 31 and 33, the driving plate 21 drives the transmission plate 4012 to rotate, which drives the end instrument 601 of the catheter 6 to bend and straighten, different rotation directions of different driving plates drive the end instrument 601 to bend in different directions. For the same rotation direction of the same driving plate, different target torques within a reasonable range of the output will drive the end instrument 601 to bend to different degrees.

For example, as shown in FIGS. 15 and 19, the corresponding degree of freedom can be adjusted by arranging the retractable wire connection relationship between the transmission plate and the catheter end. Referring to FIG. 19, the cross section of the catheter end is represented by the circumference in the figure, and the four points a-d intersecting with the circumference are connected to the transmission plates A-D by the driving wire. For example, one end of the driving wire is connected to the point a in FIG. 19, and the other end is connected to the transmission plate A, and the retractable wire of the transmission plate A is rotated, thereby driving the driving wire to pull the point a, so that the catheter end moves upward. Similarly, one end of another driving wire is connected to the point c of the circumference, and the other end is connected to the transmission plate C, and the retractable wire of the transmission plate C is rotated, thereby driving the driving wire to pull the point c, so that the catheter end moves downward. Accordingly, the transmission plates A and C can also be rotated to release the wire, and the end of the catheter can be bent upward by the transmission plate A and released by the transmission plate C, and the end of the catheter can be bent downward by the transmission plate C and released by the transmission plate A. Similarly, the end of the catheter can be bent outward or inward by the cooperation of the transmission plate B and the transmission plate D. Therefore, FIG. 19 can realize the direction control of two degrees of freedom, one degree of freedom is the up and down directions, and the other degree of freedom is the inside and outside directions. Assume that the transmission plates A and C are a pair controlling the bending in two directions on one degree of freedom, and the transmission plates B and D are a pair controlling the bending in two directions on another degree of freedom. At this time, the driving plate 1' is connected to the driving plate A, the driving plate 2' is connected to the driving plate B, the driving plate 3' is connected to the driving plate C, and the driving plate 4' is connected to the driving plate D. The mapping relationship is as follows: when bending upward, the A axis pulls the wire and the C axis releases the wire; when bending rightward, the B axis pulls the wire and the D axis releases the wire; when bending downward, the C axis pulls the wire and the A axis releases the wire; when bending leftward, the D axis pulls the wire and the B axis releases the wire. In order to more accurately adjust the end instrument 601 to a straight state (refer to FIG. 33), a suitable target torque and a suitable target rotation direction can be selected, and the driving palte 21 can be controlled to output the target torque in the target rotation direction.

Step S120, determining a position offset value of the driving plate according to a mapping relationship between an output torque of the driving plate and the position offset value, and the target torque.

In some embodiments, the position offset value is associated with the length change of the pull wire of the driving plate at the catheter end in the target bending direction. The length change can be exemplarily reflected as the position offset value of the driving plate before and after the target torque is output. In the embodiment of the present disclosure, the output torque and the position offset value can be an association relationship of a single driving plate, or an association relationship of multiple driving plates, each driving plate corresponds to an association relationship, and the association relationship of multiple driving plates can be zeroing control of each driving plate one by one, or multiple driving plates can be adjusted at one time. The mapping relationship between the output torque and the position offset value can be obtained by using kinematics and dynamics modeling, or by measuring the corresponding relationship between the output torque and the position offset value. For example, the relationship between the output torque of a single driving plate and the position offset value of the driving curved catheter end corresponding to the driving plate as shown in Table 1 is obtained. For example, in conjunction with FIG. 15, the driving plate corresponds to the transmission plate A, and the bending direction of the catheter end driven by the transmission plate A is upward bending.

**Table 1 Mapping relationship of output torque-position offset value**

| output torque | position offset value |
|---|---|
| 5N | 3mm |
| 10N | 4.6mm |
| 15N | 6.3mm |
| 20N | 7.6mm |

For example, when controlling the target torque value output by the driving plate 21 (for example, corresponding to the transmission plate A) is 10N, based on the mapping relationship between the output torque and the position offset value, it is determined that when controlling the catheter end performs bending motion in the upward bending direction, the position offset value of the driving plate 21 is 4.6mm.

Step S130, determining a target position value of the driving plate when the catheter is in a zero position state according to the current position value and the position offset value.

As an optional embodiment, determining the target position value of the driving plate when the catheter is in the zero position state based on the current position value and the position offset value includes: obtaining the difference between the current position value and the position offset value; and using the difference as the target position value of the catheter when the catheter is in the zero position state.

For example, the target torque value of the output of the driving plate 21 is 10N, at this time, the current position value obtained by the sensor detection is 65.3mm. At the same time, based on the mapping relationship between the output torque of the driving plate 21 and the position offset value, the position offset value of the driving plate 21 is determined to be 4.6mm. The current position value 65.3mm minus the position offset value 4.6mm is obtained to obtain a difference of 60.7mm. At this time, the difference 60.7mm is used as the target position value Z1 of the driving plate 21 in the zero position state.

In an optional embodiment, the installation plate includes one or more driving plates 21, for example, four driving plates 21, and steps S110-S130 need to be executed cyclically, and in the same way, target position values Z2, Z3, and Z4 of the other three driving plates 21 when the catheter is in the zero position state are obtained.

In an optional embodiment, the target position values Z1, Z2, Z3, and Z4 may be obtained by performing operations simultaneously, or may be obtained by performing operations one by one in sequence.

Step S140, controlling the driving plate to move according to the target position value to achieve the zero position state of the catheter.

In some embodiments, the driving mode of the driving plate can be changed from the torque mode to the position mode, and according to the obtained target position value Z1, the corresponding driving plate is controlled to move to the target position value, so that the zero position state of the catheter, i.e., the straight state, can be achieved. Alternatively, the current position value can be used as a starting point, and the position of the position offset value determined by the reverse motion reaches the corresponding target position value Z1, so that when the driving plate is in this position, the catheter is in the zero position state, i.e., the straight state. As an optional embodiment, if multiple driving plates are controlled to move at the same time to generate multiple target position values such as Z1, Z2, Z3, and Z4, the corresponding driving plates are controlled to move to reach the target position values respectively.

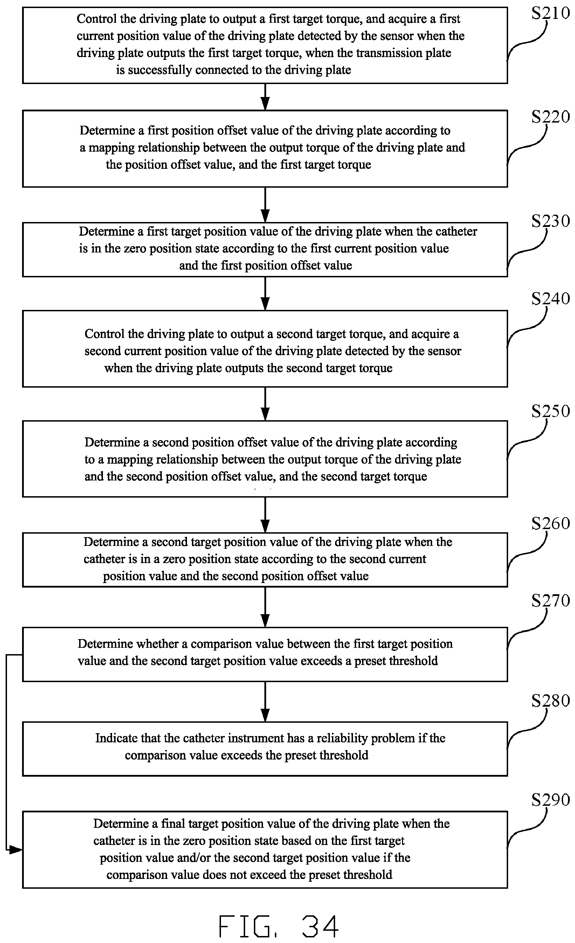

As an optional embodiment, as shown in FIG. 34, the control apparatus may also be configured to:
Step S210, control the driving plate to output a first target torque, and acquire a first current position value of the driving plate detected by the sensor when the driving plate outputs the first target torque, when the transmission plate is successfully connected to the driving plate;
Step S220, determine a first position offset value of the driving plate according to a mapping relationship between the output torque of the driving plate and the position offset value, and the first target torque;
Step S230, determine a first target position value of the driving plate when the catheter is in the zero position state according to the first current position value and the first position offset value;
Step S240, control the driving plate to output a second target torque, and acquire a second current position value of the driving plate detected by the sensor when the driving plate outputs the second target torque;
Step S250, determine a second position offset value of the driving plate according to a mapping relationship between the output torque of the driving plate and the second position offset value, and the second target torque;
Step S260, determine a second target position value of the driving plate when the catheter is in a zero position state according to the second current position value and the second position offset value;
Step S270, determine whether a comparison value between the first target position value and the second target position value exceeds a preset threshold;

The comparison value between the first target position value and the second target position value may be a difference between the two values, or a ratio between the two values.

Step S280, indicate that the catheter instrument has a reliability problem if the comparison value exceeds the preset threshold. Further, the electrical connection between the catheter instrument and the installation plate can be disconnected to prevent the catheter instrument from being used, thereby avoiding the safety risks caused by the abnormal use of the unreliable catheter instrument.

Step S290, determine a final target position value of the driving plate when the catheter is in the zero position state based on the first target position value and/or the second target position value if the comparison value does not exceed the preset threshold.

Exemplarily, the first target position value or the second target position value can be configured as the target position value; exemplary, the average value of the first target position value and the second target position value can also be configured as the target position value.

For example, controlling the first target torque value output by the driving plate 21 is 10N, at this time, the first current position value obtained by the sensor detection is 65.3 mm, based on the mapping relationship between the output torque of the driving plate 21 and the position offset value, the first position offset value of the driving plate 21 is determined to be 4.6 mm, the first current position value 65.3 mm minus the first position offset value 4.6 mm is obtained to obtain the first target position value 60.7 mm, controlling the second target torque value output by the driving plate 21 is 15N, at this time, the second current position value obtained by the sensor detection is 67.1 mm, based on the mapping relationship between the output torque of the driving plate 21 and the position offset value, it is determined that the second position offset value of the driving plate 21 is 6.3 mm, and the second current position value 67.1 mm minus the second position offset value 6.3 mm is used to obtain a first target position value 60.8 mm, it is determined that the mutual comparison values of 60.7 mm and 60.8 are less than 1 mm, indicating that the catheter instrument is reliable, at this time, for example, the difference 60.7 mm can be used as the target position value Z1 of the driving plate 21 in the zero position state, and the motion of the driving plate is controlled to achieve the zero position state of the driving plate.

The technical solution of the disclosed embodiment determines the target position value of the driving plate when the catheter is in a zero-position state by acquiring the current position value and the position offset value, and controls the motion of the driving plate to achieve the zero-position state, it can automatically perform zeroing without manual operation, thereby improving zeroing efficiency and accuracy.

In some embodiments, described control apparatus is also configured to be used for: detect whether transmission plate is successfully connected with driving plate. When the internal tube instrument 5 or the external sheath instrument 4 are installed in first manipulator assembly 3 or second manipulator assembly 2, first carry out position-finding operation, i.e. the driving plate 21 is aligned with transmission plate 4012 and connected, and system can detect whether transmission plate 4012 is successfully connected with driving plate 21. As shown in FIG. 31, the installation plate includes one or more driving plates 21, for example, four driving plates 21, and the catheter instrument includes one or more transmission plates 4012, for example, four transmission plates 4012, the number of the driving plates 21 matches the number of the transmission plates 4012, for example, the installation plate includes four driving plates 21, and the catheter instrument also includes four transmission plates 4012. When the catheter instrument is installed on the installation plate, a positioning operation is first performed, that is, the transmission plate 4012 is paired and connected with the driving plate 21, and a zeroing operation is performed after successful assembly.

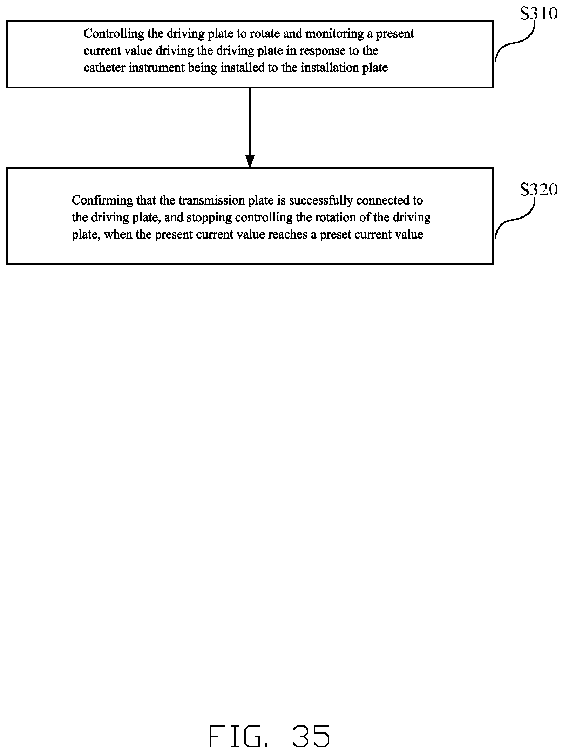

As shown in FIG. 35, the step of detecting whether the transmission plate is successfully connected to the driving plate, includes:
Step S310, controlling the driving plate to rotate and monitoring a present current value driving the driving plate in response to the catheter instrument being installed to the installation plate.

In some embodiments, a sensor installed at the end of the installation plate, such as an electronic identification device, senses that the catheter instrument has been installed and sends a signal to the control device. After receiving the signal, the control apparatus controls the driving plate 21 to rotate. At this time, the transmission plate 4012 presses down the driving plate 21, and there is no matching connection, the transmission plate 4012 includes two protrusions, and the driving plate 21 includes two grooves. The protrusions are not matched and connected into the grooves, and the driving plate 21 needs to be controlled to rotate until the protrusions are matched and connected into the grooves. Since the protrusions press down the driving plate during the rotation process, there is friction between the two, and a very large current is not required to overcome the friction. When the protrusions are matched into the grooves, there will be a thrust to push the transmission plate to rotate, the thrust is obviously greater than the friction, and a larger current is required to overcome the thrust. Therefore, it is possible to determine whether the protrusions have been matched and connected into the grooves, that is, whether the transmission plate 4012 and the driving plate 21 are successfully connected, by monitoring the present current value of the driving plate 21.

Step S320, confirming that the transmission plate is successfully connected to the driving plate, and stopping controlling the rotation of the driving plate, when the present current value reaches a preset current value.

In some embodiments, during the rotation of the driving plate 21, the current value of the current driving the driving plate 21 is constantly monitored. When the current value reaches a preset range, for example, greater than 10 mA, it indicates that the protrusion is matched and connected into the groove. At this time, the friction force is reduced and thrust is generated, so a higher current value is required to drive the rotation. At this time, it is determined that the transmission plate 4012 is successfully connected to the driving plate 21, and the control of the rotation of the driving plate 21 is stopped.

Further, when the present current value does not reach the preset current value, determining that the transmission plate and the driving plate are not successfully connected, and continuously controlling the driving plate to rotate.

In some embodiments, monitoring the present current value driving the driving plate 21 is a continuous monitoring process, when the current value reaches the preset current value, it indicates that the protrusion matches the groove, generating thrust, and a larger current is required. The driving plate 21 is controlled to stop rotating, when the current value does not reach the preset current value, it indicates that the protrusion is rotating by rubbing against the edge of the groove, generating friction, and does not require too much current, the driving plate 21 is continued to be controlled to rotate until the protrusion matches the groove, and the transmission plate 4012 is successfully connected to the driving plate 21.

As an optional embodiment, controlling the driving plate to rotate includes: controlling the driving plate to rotate alternately in a clockwise direction and a counterclockwise direction, wherein the range of rotation of the driving plate in the clockwise direction is between [0°, 180°], and the range of rotation of the driving plate in the counterclockwise direction is between [-180°, 0°].

By controlling the rotation range of the driving plate, it is possible to prevent the instrument from exceeding the physical limit and being damaged due to single-direction rotation, thereby increasing the service life of the instrument.

In some embodiments, the driving plate includes a motor and a driving plate driven to rotate by the motor, and the driving plate also includes an elastic element, which is coupled to the driving plate, with the help of the elastic element, the driving plate can float in the elastic deformation direction of the elastic element. The elastic element can be, for example, an element with elastic properties such as a spring, which is located below the driving plate, when the transmission plate presses down the driving plate, it can float in the elastic deformation direction of the elastic element to achieve a tight abutment of the concave and convex fit.

In some embodiments, the transmission plate and the driving plate are removably connected by a concave-convex fit. Specifically, one of the driving plate and the transmission plate includes a connecting column, and the other includes a connecting groove, the connecting column is adapted to the connecting groove, the transmission plate can be the connecting column and the driving plate can be the connecting groove, or the transmission plate can be the connecting groove and the driving plate can be the connecting column, the embodiments of the present disclosure do not limit this. In this embodiment, the number of connecting columns of the transmission plate is two, and the number of connecting grooves of the driving plate is two. It can be understood that the number of connecting columns or connecting grooves can be set to three or four as needed, etc., and the embodiments of the present disclosure do not limit this.

Further, one of the driving plate and the transmission plate includes the connecting column, and the other includes the connecting groove, the connecting column is adapted to the connecting groove, and the top of the connecting column is configured as an arc structure. Specifically, the top of the connecting column is configured as an arc structure, which mainly has the following functions: 1. Better matching with the motor connector; 2. Reducing friction resistance during positioning, facilitating observation of the current value of the driving plate. More specifically, the top of the connecting column can also be configured as a spherical structure, which is not limited in the embodiments of the present disclosure.

The disclosed embodiment also provides a control method of catheter robot, the catheter robot includes: an installation plate, including a driving plate, and a sensor for detecting the position of the driving plate; a catheter instrument, including a catheter, and a transmission plate for driving the distal end of the catheter to move, the transmission plate being removably connected to the driving plate;

As shown in FIG. 32, the control method includes the following steps:
Step S110: controlling the driving plate to output a target torque, and acquiring a current position value of the driving plate detected by the sensor when the driving plate outputs the target torque, when the transmission plate is successfully connected to the driving plate;
Step S120, determining a position offset value of the driving plate according to a mapping relationship between an output torque of the driving plate and the position offset value, and the target torque;
Step S130, determining a target position value of the driving plate when the catheter is in a zero position state according to the current position value and the position offset value;
Step S140, controlling the driving plate to move according to the target position value to achieve the zero position state of the catheter.

Further, controlling the driving plate to output the target torque, by:
determining a target rotation direction in which the drive plate pulls the end of the catheter to bend through the transmission plate according to the mapping relationship between the rotation direction of the driving plate and a bend direction of the end of the catheter;
controlling the driving plate to output the target torque in the target rotation direction.

Further, determining the target position value of the driving plate when the catheter is in the zero position state according to the current position value and the position offset value, by:
acquiring a difference between the current position value and the position offset value;
using the difference as the target position value of the catheter in the zero position state.

Further, the control apparatus is further configured to:
detect whether the transmission plate is successfully connected to the driving plate.

Further, as shown in FIG. 35, detecting whether the transmission plate is successfully connected to the driving plate, includes:
Step S310, controlling the driving plate to rotate and monitoring a present current value driving the driving plate in response to the catheter instrument being installed to the installation plate;
Step S320, confirming that the transmission plate is successfully connected to the driving plate, and stopping controlling the rotation of the driving plate, when the present current value reaches a preset current value; or,
when the present current value does not reach the preset current value, determining that the transmission plate and the driving plate are not successfully connected, and continuously controlling the driving plate to rotate.

Further, controlling the driving plate to rotate, includes:
controlling the driving plate to rotate includes: controlling the driving plate to rotate alternately in a clockwise direction and a counterclockwise direction, wherein the range of rotation of the driving plate in the clockwise direction is between [0°, 180°], and the range of rotation of the driving plate in the counterclockwise direction is between [-180°, 0°].

The technical solution of the embodiment of the present disclosure determines the target position value of the driving plate when the catheter is in the zero position state by obtaining the current position value and the position offset value, and controls the motion of the driving plate to achieve the zero position state, no manual operation is required, and zero adjustment can be performed automatically, thereby improving the efficiency and accuracy of zero adjustment.

The present disclosure also provides a storage medium containing computer executable instructions, wherein the computer executable instructions are used to execute a control method of catheter robot when executed by a computer processor, the method includes:
controlling the driving plate to output a target torque, and acquire a current position value of the driving plate detected by the sensor when the driving plate outputs the target torque, when the transmission plate is successfully connected to the driving plate;
determining a position offset value of the driving plate according to a mapping relationship between an output torque of the driving plate and the position offset value, and the target torque;
determining a target position value of the driving plate when the catheter is in a zero position state according to the current position value and the position offset value;
controlling the driving plate to move according to the target position value to achieve the zero position state of the catheter.

Of course, the storage medium containing computer executable instructions provided in the embodiments of the present disclosure, whose computer executable instructions are not limited to the method operations described above, can also execute related operations in the control method of the catheter robot provided in any embodiment of the present disclosure.

The computer-readable storage medium of the disclosed embodiment can adopt any combination of one or more computer-readable media. The computer-readable medium can be a computer-readable signal medium or a computer-readable storage medium. The computer-readable storage medium can be, for example, but not limited to, a system, device or component of electricity, magnetism, light, electromagnetic, infrared, or semiconductor, or any combination of the above. More specific examples of computer-readable storage media (a non-exhaustive list) include: an electrical connection with one or more conductors, a portable computer disk, a hard disk, a random access memory (RAM), a read-only memory (ROM), an erasable programmable read-only memory (EPROM or flash memory), an optical fiber, a portable compact disk read-only memory (CD-ROM), an optical storage device, a magnetic storage device, or any suitable combination of the above. In this document, a computer-readable storage medium can be any tangible medium that contains or stores a program that can be used by or in conjunction with an instruction execution system, apparatus, or device.

Computer-readable signal media may include a data signal propagated in baseband or as a carrier wave part, wherein a computer-readable program code is carried. The data signal of this propagation may take various forms, including but not limited to electromagnetic signals, optical signals, or any suitable combination thereof. Computer-readable signal media may also be any computer-readable medium other than a computer-readable storage medium, which may send, propagate, or transmit a program used by an instruction execution system, an apparatus, or a device or used in combination therewith.

The program code contained on the storage medium can be transmitted using any appropriate medium, including but not limited to wireless, wire, optical cable, RF, etc., or any suitable combination of the above.

Computer program code for performing operations of the present disclosure may be written in one or more programming languages, or a combination thereof, including object-oriented programming languages such as Java, Smalltalk, C++, and conventional procedural programming languages such as "C" language or similar programming languages. Program code can be executed completely on user's computer, partially on user's computer, executed as an independent software package, partially on user's computer and partially on remote computer, or completely on remote computer or terminal. In the case of involving remote computer, remote computer can be connected to user's computer through any kind of network, including local area network (LAN) or wide area network (WAN), or can be connected to external computer (for example, using Internet service provider to connect through Internet).

The technical solution of the embodiment of the present disclosure determines the target position value of the drive disk when the catheter is in the zero position state by obtaining the current position value and the position offset value, and controls the movement of the drive disk to achieve the zero position state. No manual operation is required, and zero adjustment can be performed automatically, thereby improving the efficiency and accuracy of zero adjustment.

## Claims

1. A catheter robot, comprising:
a manipulator assembly, the manipulator assembly configured to install and drive a catheter instrument; and
a control apparatus, the control apparatus coupled with the manipulator assembly and configured to:
acquire an installation direction of the catheter instrument in the manipulator assembly;
determine a target mapping relationship according to the installation direction;
wherein the target mapping relationship comprises a first mapping relationship and a second mapping relationship, the first mapping relationship comprises a mapping relationship between a driving mechanism in the manipulator assembly and a target motion direction when the installation direction being a first direction is associated, the second mapping relationship comprises a mapping relationship between the driving mechanism in the manipulator assembly and the target motion direction when the installation direction being a second direction is associated;
control, based on the target mapping relationship, the driving mechanism in the manipulator assembly to manipulate a motion of the catheter instrument in the target motion direction.

2. The catheter robot according to claim 1, wherein the manipulator assembly comprises a first manipulator assembly and a second manipulator assembly, the catheter instrument comprises an internal tube instrument and an external sheath instrument, acquiring the installation direction of the catheter instrument in the manipulator assembly, comprises:
determining the installation direction as the first direction when the internal tube instrument installed to the first manipulator assembly towards the first direction and the external sheath instrument installed to the second manipulator assembly towards the first direction are detected;
determining the installation direction as the second direction when the external sheath instrument installed to the first manipulator assembly towards the second direction and the internal tube instrument installed to the second manipulator assembly towards the second direction are detected;
the first direction is a direction of the first manipulator assembly adjacent to a side of the second manipulator assembly, the second direction is a direction of the first manipulator assembly away from the side of the second manipulator assembly.

3. The catheter robot according to claim 2, wherein
the first mapping relationship comprises: configuring a direction of the driving mechanism in the second manipulator assembly manipulating a forward feed motion of the external sheath instrument, and a direction of the driving mechanism in the first manipulator assembly manipulating a forward feed motion of the internal tube instrument to be the same as the first direction;
the second mapping relationship comprises: configuring a direction of the driving mechanism in the first manipulator assembly manipulating a forward feed motion of the external sheath instrument, and a direction of the driving mechanism in the second manipulator assembly manipulating a forward feed motion of the internal tube instrument to be the same as the second direction.

4. The catheter robot according to claim 3, wherein
the first mapping relationship comprises: configuring the second manipulator assembly as a main manipulator assembly, and the first manipulator assembly as a vice manipulator assembly; configuring the vice manipulator assembly to move synchronously with the main manipulator assembly;
the second mapping relationship comprises: configuring the first manipulator assembly as the main manipulator assembly, and the second manipulator assembly as the vice manipulator assembly; configuring the vice manipulator assembly to move synchronously with the main manipulator assembly.

5. The catheter robot according to any one of claims 2 to 4, wherein the first manipulator assembly comprises a first installation plate for connecting the catheter instrument, the second manipulator assembly comprises a second installation plate for connecting the catheter instrument,
the first mapping relationship comprises:
configuring a first driving mechanism in the first installation plate to drive the internal tube instrument to manipulate the internal tube instrument to move in a first bending direction, and configuring a second driving mechanism to drive the internal tube instrument to manipulate the internal tube instrument to move in a second bending direction;
configuring a third driving mechanism in the second installation plate to drive the external sheath instrument to manipulate the external sheath instrument to move in the first bending direction, and configuring a fourth driving mechanism to drive the external sheath instrument to manipulate the external sheath instrument to move in the second bending direction;
the second mapping relationship comprises:
configuring the first driving mechanism in the first installation plate to drive the external sheath instrument to manipulate the external sheath instrument to move in the first bending direction, and configuring the second driving mechanism to drive the external sheath instrument to manipulate the external sheath instrument to move in the second bending direction;
configuring the third driving mechanism in the second installation plate to drive the internal tube instrument to manipulate the internal tube instrument to move in the first bending direction, and configuring the fourth driving mechanism to drive the internal tube instrument to manipulate the internal tube instrument to move in the second bending direction.

6. The catheter robot according to any one of claims 2 to 4, wherein after determining the target mapping relationship according to the installation direction, the control apparatus is further configured to:
if the mapping relationship is the first mapping relationship, detect whether the first manipulator assembly is installed with the internal tube instrument, and whether the second manipulator assembly is installed with the external sheath instrument;
when the first manipulator assembly is not installed with the internal tube instrument, or the second manipulator assembly is not installed with the external sheath instrument, control the catheter robot to raise an alarm.

7. The catheter robot according to any one of claims 2 to 4, wherein after determining the target mapping relationship according to the installation direction, the control apparatus is further configured to:
if the mapping relationship is the second mapping relationship, detect whether the first manipulator assembly is installed with the external sheath instrument, and whether the second manipulator assembly is installed with the internal tube instrument;
when the first manipulator assembly is not installed with the external sheath instrument, or the second manipulator assembly is not installed with the internal tube instrument, control the catheter robot to raise an alarm.

8. The catheter robot according to claim 5, wherein after determining the target mapping relationship according to the installation direction, the control apparatus is further configured to:
if the mapping relationship is the first mapping relationship, when it is detected that the internal tube instrument is installed on the first manipulator assembly toward the first direction, and when it is detected that the external sheath instrument is installed on the second manipulator assembly toward the second direction, control the second installation plate to rotate so that the external sheath instrument faces the first direction.

9. The catheter robot according to claim 5, wherein after determining the target mapping relationship according to the installation direction, the control apparatus is further configured to:
if the mapping relationship is the first mapping relationship, when it is detected that the internal tube instrument is installed on the first manipulator assembly toward the second direction, and when it is detected that the external sheath instrument is installed on the second manipulator assembly toward the first direction, control the second installation plate to rotate so that the internal tube instrument faces the second direction.

10. The catheter robot according to claim 5, wherein after determining the target mapping relationship according to the installation direction, the control apparatus is further configured to:
if the mapping relationship is the second mapping relationship, when it is detected that the external sheath instrument is installed on the first manipulator assembly toward the second direction, and when it is detected that the internal tube instrument is installed on the second manipulator assembly toward the first direction, control the second installation plate to rotate so that the internal tube instrument faces the second direction.

11. The catheter robot according to claim 5, wherein after determining the target mapping relationship according to the installation direction, the control apparatus is further configured to:
if the mapping relationship is the second mapping relationship, when it is detected that the external sheath instrument is installed on the first manipulator assembly toward the first direction, and when it is detected that the internal tube instrument is installed on the second manipulator assembly toward the second direction, control the first installation plate to rotate so that the external sheath instrument faces the second direction.

12. The catheter robot according to claim 5, wherein the control apparatus is further configured to: after detecting that the catheter instrument is removed, control the first installation plate and/or the second installation plate to rotate to an initial position.

13. The catheter robot according to any one of claims 2 to 4, wherein an installation direction detection instrument is provided between the manipulator assembly and the catheter instrument, acquiring the installation direction of the catheter instrument in the manipulator assembly by:
detecting the installation direction of the catheter instrument in the manipulator assembly through the installation direction detection instrument.

14. The catheter robot according to claim 13, wherein the installation direction detection instrument comprises: a first interface and a second interface are installed on the manipulator assembly and an alignment interface is installed on the catheter device, the alignment interface selectively cooperates with the first interface and the second interface.

15. The catheter robot according to any one of claims 2 to 4, wherein the catheter robot is equipped with an installation direction input module, acquiring the installation direction of the catheter instrument in the manipulator assembly, by:
acquiring the installation direction of the catheter instrument in the manipulator assembly through the installation direction input module.

16. The catheter robot according to claim 1, wherein
the manipulator assembly comprises an installation plate, the installation plate comprises a driving plate and a sensor, the sensor is configured to detect a position of the driving plate;
the catheter instrument comprises a catheter and a transmission plate, the transmission plate is configured to drive an end of the catheter to move, the transmission plate is detachably connected to the driving plate;
the control apparatus is configured to:
control the driving plate to output a target torque, and acquire a current position value of the driving plate detected by the sensor when the driving plate outputs the target torque, when the transmission plate is successfully connected to the driving plate;
determine a position offset value of the driving plate according to a mapping relationship between an output torque of the driving plate and the position offset value, and the target torque;
determine a target position value of the driving plate when the catheter is in a zero position state according to the current position value and the position offset value;
control the driving plate to move according to the target position value to achieve the zero position state of the catheter.

17. The catheter robot according to claim 16, wherein controlling the driving plate to output the target torque, by:
determining a target rotation direction in which the drive plate pulls the end of the catheter to bend through the transmission plate according to the mapping relationship between the rotation direction of the driving plate and a bend direction of the end of the catheter;
controlling the driving plate to output the target torque in the target rotation direction.

18. The catheter robot according to claim 16, wherein determining the target position value of the driving plate when the catheter is in the zero position state according to the current position value and the position offset value, by:
acquiring a difference between the current position value and the position offset value;
using the difference as the target position value of the catheter in the zero position state.

19. A control method of catheter robot, the catheter robot comprising:
a manipulator assembly, the manipulator assembly configured to install and drive a catheter instrument; and
a control apparatus, the control apparatus coupled with the manipulator assembly and configured to:
acquire an installation direction of the catheter instrument in the manipulator assembly;
determine a target mapping relationship from one of a first mapping relationship and a second mapping relationship according to the installation direction, the target mapping relationship comprises a first mapping relationship and a second mapping relationship, the first mapping relationship comprises a mapping relationship between a driving mechanism in the manipulator assembly and a target motion direction when the installation direction being a first direction is associated, the second mapping relationship comprises a mapping relationship between the driving mechanism in the manipulator assembly and the target motion direction when the installation direction being a second direction is associated;
control, based on the target mapping relationship, the driving mechanism in the manipulator assembly to manipulate a motion of the catheter instrument in the target motion direction.

20. A readable storage medium, a computer program is stored on the readable storage medium, and when the computer program is executed by a processor, the steps of the control method of catheter robot according to claim 19 are implemented.
